(19) Europäisches Patentamt
European Patent Office
Office européen des brevets

(11) Publication number: 0 549 365 B1

(12) EUROPEAN PATENT SPECIFICATION

(45) Date of publication of patent specification: 09.08.95

(51) Int. Cl.6: C07D 277/34, A61K 31/425

(21) Application number: 92311813.7

(22) Date of filing: 24.12.92

# (54) Thiazolidine compounds, their preparation and their therapeutic uses.

(30) Priority: 26.12.91 JP 344571/91

(43) Date of publication of application:
30.06.93 Bulletin 93/26

(45) Publication of the grant of the patent:
09.08.95 Bulletin 95/32

(84) Designated Contracting States:
AT BE CH DE DK ES FR GB GR IE IT LI LU MC NL PT SE

(56) References cited:
EP-A- 0 008 203
EP-A- 0 139 421
EP-A- 0 208 420
EP-A- 0 441 605

(73) Proprietor: Sankyo Company Limited
5-1 Nihonbashi Honcho 3-chome
Chuo-ku
Tokyo (JP)

(72) Inventor: Yoshioka, Takao, c/o Sankyo Company Limited
2-58, Hiromachi 1-chome,
Shinagawa-ku
Tokyo (JP)
Inventor: Nishi, Takahide, c/o Sankyo Company Limited
2-58, Hiromachi 1-chome,
Shinagawa-ku
Tokyo (JP)
Inventor: Kanai Tsutomu, c/o Sankyo Company Limited
2-58, Hiromachi 1-chome,
Shinagawa-ku
Tokyo (JP)
Inventor: Aizawa, Yuichi, c/o Sankyo Company Limited
2-58, Hiromachi 1-chome,
Shinagawa-ku
Tokyo (JP)
Inventor: Wada, Kunio, c/o Sankyo Company Limited
2-58, Hiromachi 1-chome,
Shinagawa-ku
Tokyo (JP)
Inventor: Fujita, Takashi, c/o Sankyo Company Limited
2-58, Hiromachi 1-chome,
Shinagawa-ku
Tokyo (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **Horikoshi, Hiroyoshi, c/o Sankyo Company Limited**
**2-58, Hiromachi 1-chome,**
**Shinagawa-ku**
**Tokyo (JP)**

(74) Representative: **Gibson, Christian John Robert et al**
**MARKS & CLERK,**
**57/60 Lincoln's Inn Fields**
**London WC2A 3LS (GB)**

## Description

The present invention relates to a series of thiazolidine derivatives which are characterised by the presence, inter alia, of a hydroquinone group or naphthohydroquinone group in their molecules. These compounds have valuable therapeutic and prophylactic activities, including anti-diabetic activities, and the invention therefore also provides methods and compositions using these compounds for the treatment and prophylaxis of diabetes and diabetic complications, as described in greater detail hereafter. The invention also provides processes for preparing these novel compounds.

A number of compounds in which a substituted alkoxybenzyl group is attached to the 5-position of a thiazolidine-2,4-dione group is known. These compounds can be generally represented by the formula (A):

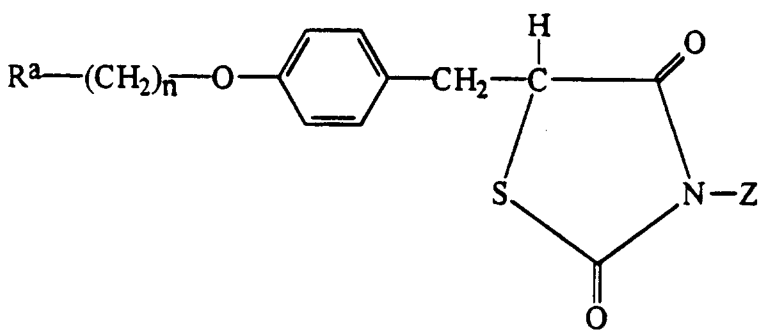

(A)

For example, European Patent Publication No. 8 203 discloses a series of compounds of the type shown in formula (A) in which $R^a$ may be an alkyl or cycloalkyl group. European Patent Publication No. 139 421 discloses such compounds in which the group equivalent to $R^a$ in formula (A) above is a chroman or similar group, and Y. Kawamatsu et al. [Chem. Pharm. Bull., 30, 3580 - 3600 (1982)] disclose a wide range of such compounds of formula (A) in which $R^a$ may be various phenyl, substituted phenyl, alkylamino, cycloalkyl, terpenyl and heterocyclic groups.

EP 208 420 also describes thiazolidine derivatives generally represented by formula (A), in which $R^a$ may be a heterocyclic group, which compounds are suitable as therapeutic agents for diabetes and hyperlipemia.

All of the prior thiazolidine derivatives referred to above are said to have the ability to lower blood glucose levels, and it is thought that this is achieved by reducing insulin resistance in the peripheral system.

However, it is currently thought that the compounds of the prior art which are closest to those of the present invention are disclosed in European Patent Publication No. 441 605, as these, like the compounds of the present invention may contain a hydroquinone group or naphthohydroquinone group, although attached in a different manner to the alkylene group of formula $-(CH_2)_n-$.

We have now discovered a series of novel compounds which, in addition to the ability to reduce insulin resistance in the peripheral tissues (which is the sole basis of the antidiabetic activity of most of the prior art compounds) also exhibits other activities, for example, like the compounds of European Patent Publication No. 441 605, the present compounds have the ability to suppress hepatic gluconeogenesis in the liver, which is one of the causes of diabetes. These additional activities, combined with a low toxicity, mean that the compounds of the present invention will be more effective than the prior art compounds and able to treat a wider range of disorders. The compounds of the present invention have been surprisingly found to have a substantially better activity than do the compounds of prior art European Patent Publication No. 441 605.

Accordingly, the compounds of the present invention are those compounds of formula (I):

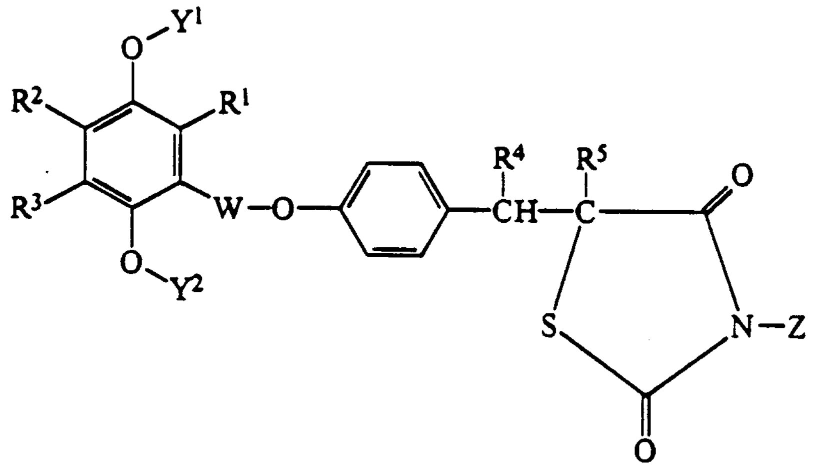

(I)

in which:

$R^1$ represents an alkyl group having from 1 to 5 carbon atoms;

$R^2$ and $R^3$ are the same or different and each represents an alkyl group having from 1 to 5 carbon atoms or an alkoxy group having from 1 to 5 carbon atoms,

or

$R^2$ and $R^3$ together form a benzene ring which is unsubstituted or which is substituted by at least one substituent selected from substituents A, defined below, and, when $R^2$ and $R^3$ together form said benzene ring, $R^1$ represents a hydrogen atom, a halogen atom or an alkyl group having from 1 to 5 carbon atoms;

$R^4$ and $R^5$ both represent hydrogen atoms;

$Y^1$ and $Y^2$ are the same as each other or different from each other, and each represents:

a hydrogen atom,

an alkyl group having from 1 to 5 carbon atoms,

an aliphatic carboxylic acyl group having from 1 to 7 carbon atoms, or

a benzoyl, naphthoyl, pyridinecarbonyl or quinolinecarbonyl group which is unsubstituted or is substituted by at least one of substituents A, defined below;

W represents a single bond or an alkylene group having from 1 to 5 carbon atoms; and

Z represents a hydrogen atom or a 1/x equivalent of a cation, where x is the charge on the cation; and

substituents A are selected from alkyl groups having from 1 to 5 carbon atoms, alkoxy groups having from 1 to 5 carbon atoms and halogen atoms.

The invention also provides a pharmaceutical composition for the treatment or prophylaxis of diabetes or hyperlipemia, which comprises an effective amount of an active compound in admixture with a pharmaceutically acceptable carrier or diluent, in which said active compound is selected from compounds of formula (I), defined above.

The invention still further provides the use of compounds of formula (I), defined above, in therapy.

The invention still further provides the use of compounds of formula (I), defined above, for the manufacture of a medicament for the treatment or prophylaxis of diabetes or hyperlipemia in a mammal, which may be human.

The invention also provides processes for the preparation of the compounds of the present invention, which processes are described in more detail hereafter.

In the compounds of the present invention, where $R^1$, $R^2$, $R^3$, $Y^1$, $Y^2$ or substituent A represents an alkyl group, this may be a straight or branched chain alkyl group having from 1 to 5 carbon atoms, and examples include the methyl, ethyl, propyl, isopropyl, butyl, isobutyl, sec-butyl, t-butyl, pentyl, neopentyl and isopentyl groups. Of these, we prefer those alkyl groups having from 1 to 4 carbon atoms, most preferably the methyl group.

Where $R^2$ and $R^3$ together form a benzene ring (that is, the benzene ring forms, with the ring to which it is fused, a naphthohydroquinone group), this may be unsubstituted or it may be substituted, on the ring portion represented by $R^2$ and $R^3$, by one or more of substituents A, as exemplified below. In addition, in this case, $R^1$, may represent a hydrogen atom, a halogen atom, or one of the alkyl groups exemplified

above. Also, in this case, substituents A may be selected from alkyl groups having from 1 to 5 carbon atoms, such as those exemplified above, alkoxy groups having from 1 to 5 carbon atoms and halogen atoms.

Where the resulting fused benzene ring is substituted, there is no particular limitation on the number of substituents, except such as may be imposed by the number of substitutable positions or possibly by steric constraints. In general, where the group is substituted, from 1 to 4 substituents are possible, although fewer are preferred, from 1 to 3 being generally more preferred, and 1 or 2 being still more preferred. We most prefer no substituents on this fused benzene ring.

Where $R^2$, $R^3$ or substituent A represents an alkoxy group, this may be a straight or branched chain alkoxy group having from 1 to 5 carbon atoms, and examples include the methoxy, ethoxy, propoxy, isopropoxy, butoxy, isobutoxy, sec-butoxy, t-butoxy, pentyloxy, neopentyloxy and isopentyloxy groups. Of these, we prefer those alkoxy groups having from 1 to 4 carbon atoms, most preferably the methoxy group.

Where $R^1$ or substituent A represents a halogen atom, this may be, for example, a chlorine, fluorine or bromine atom, preferably a chlorine or fluorine atom, and most preferably a chlorine atom.

Where $Y^1$ and/or $Y^2$ represents an aliphatic carboxylic acyl group having from 1 to 7 carbon atoms, this may be a straight or branched chain group, and examples of such acyl groups include the formyl, acetyl, propionyl, butyryl, isobutyryl, valeryl, isovaleryl, pivaloyl, pentanoyl and hexanoyl groups. Of these, we prefer those straight or branched chain alphatic carboxylic acyl groups having from 2 to 4 carbon atoms, and most prefer the acetyl group.

Where $Y^1$ and/or $Y^2$ represents an optionally substituted benzoyl, naphthoyl, pyridinecarbonyl or quinolinecarbonyl group, examples of such groups include the benzoyl, $\alpha$-naphthoyl, $\beta$-naphthoyl, picolinoyl, nicotinoyl, isonicotinoyl, quinoline-2-carbonyl, quinoline-3-carbonyl and quinoline-4-carbonyl groups. Of these, we prefer an optionally substituted benzoyl group or an optionally substituted pyridinecarbonyl group, and most prefer a nicotinoyl group.

W may represent a single bond or an alkylene group. Where W represents an alkylene group, this may be a straight or branched chain alkylene group having from 1 to 5 carbon atoms. The bonds of the alkylene group by which it is attached, on the one hand, to the hydroquinone or naphthohydroquinone group and, on the other hand, to the oxygen atom may be on the same carbon atom or on different carbon atoms. Where the bonds are on the same carbon atom, the groups are sometimes referred to as "alkylidene groups". It is, however, conventional to use the general term "alkylene group" to include both those groups where the bonds are on the same carbon atom and those where they are on different carbon atoms. Examples of such groups include the methylene, ethylene, trimethylene, tetramethylene, pentamethylene, methylmethylene, 2,2-dimethyltrimethylene, 2-ethyltrimethylene, 1-methyltetramethylene, 2-methyltetramethylene and 3-methyltetramethylene groups, of which we prefer those alkylene groups (which may be straight or branched chain groups) having from 1 to 4 carbon atoms, and most prefer the straight chain alkylene groups having 2 or 3 carbon atoms.

Z may represent a hydrogen atom or a cation. Where the cation has a plural charge, for example 2+, then Z represents a number of equivalents of that cation which is the reciprocal of that charge. For example, where Z represents an alkali metal, examples of such alkali metals include lithium, sodium or potassium, and the charge borne by these metals being 1+, Z represents, for each equivalent of the compound of formula (I), one equivalent of the metal. Where Z represents an alkaline earth metal, examples of such alkaline earth metals include calcium or barium, and the charge borne by these metals being 2+, Z represents, for each equivalent of the compound of formula (I), one half equivalent of the metal. Where Z represents a basic amino acid, examples of such amino acids include lysine or arginine, and the charge borne by these acids being 1+, Z represents, for each equivalent of the compound of formula (I), one equivalent of the acid.

Preferably Z represents an alkali metal, one half equivalent of an alkaline earth metal or a basic amino acid.

The compounds of the present invention necessarily contain at least one asymmetric carbon atom at the 5-position of the thiazolidine ring, and, depending on the nature of the groups and atoms represented by $R^1$, $R^2$, $R^3$, $Y^1$, $Y^2$ and W, may contain several asymmetric carbon atoms in their molecules. They can thus form optical isomers. They can also form tautomers due to the interconversion of the imide group formed by the nitrogen atom at the 3-position of the thiazolidine ring and the oxo groups at the 2- and 4-positions of the thiazolidine ring to a group of formula -N=C(OH)-. Although these optical isomers and tautomers are all represented herein by a single molecular formula, the present invention includes both the individual, isolated isomers and mixtures, including racemates, thereof. Where stereospecific synthesis techniques are employed or optically active compounds are employed as starting materials, individual isomers may be prepared directly; on the other hand, if a mixture of isomers is prepared, the individual isomers may be

obtained by conventional resolution techniques.

A preferred class of compounds of the present invention are those compounds of formula (I) in which:

$R^1$ represents an alkyl group having from 1 to 5 carbon atoms;

$R^2$ and $R^3$ are the same or different (particularly preferably the same) and each represents an alkyl group having from 1 to 5 carbon atoms or an alkoxy group having 1 to 5 carbon atoms, or $R^2$ and $R^3$ together form a benzene ring which is unsubstituted or which is substituted by at least one of substituents A, defined above, and, when $R^2$ and $R^3$ together form said benzene ring, $R^1$ represents a hydrogen atom, a halogen atom or an alkyl group having from 1 to 5 carbon atoms;

$R^4$ and $R^5$ each represents a hydrogen atom;

$Y^1$ and $Y^2$ are the same and each represents a hydrogen atom, a methyl group, an acetyl group, a benzoyl group or a nicotinoyl group;

W represents an alkylene group having from 1 to 5 carbon atoms; and

Z represents a hydrogen atom or a sodium atom.

A more preferred class of compounds of the present invention are those compounds of formula (I) in which:

$R^1$ represents an alkyl group having from 1 to 5 carbon atoms;

$R^2$ and $R^3$ are the same and each represents an alkyl group having from 1 to 5 carbon atoms, or $R^2$ and $R^3$ together form an unsubstituted benzene ring, and, when $R^2$ and $R^3$ together form said benzene ring, $R^1$ represents a hydrogen atom, a methyl group or a chlorine atom, more preferably a hydrogen atom;

$R^4$ and $R^5$ each represents a hydrogen atom;

$Y^1$ and $Y^2$ are the same and each represents a hydrogen atom, a methyl group or an acetyl group, more preferably a methyl group or an acetyl group;

W represents an alkylene group having from 2 to 4 carbon atoms; and

Z represents a hydrogen atom or a sodium atom.

The most preferred class of compounds of the present invention are those compounds of formula (I) in which:

$R^1$, $R^2$ and $R^3$ each represents a methyl group;

$Y^1$ and $Y^2$ are the same and each represents a methyl or acetyl group;

W represents an ethylene or trimethylene group; and

Z represents a hydrogen atom or a sodium atom.

Specific examples of compounds of the invention are those compounds having the following formulae (I-1) to (I-2), in which the substituents are as defined in the respective one of Tables 1 to 2, i.e. Table 1 relates to formula (I-1), Table 2 relates to formula (I-2). In the Tables the following abbreviations are used for certain groups;

otherwise, standard internationally recognised symbols are used to designate atoms:

| | |
|---|---|
| Ac | acetyl |
| Boz | benzoyl |
| Bu | butyl |
| Byr | butyryl |
| Et | ethyl |
| Me | methyl |
| Nic | nicotinoyl |
| iPr | isopropyl |

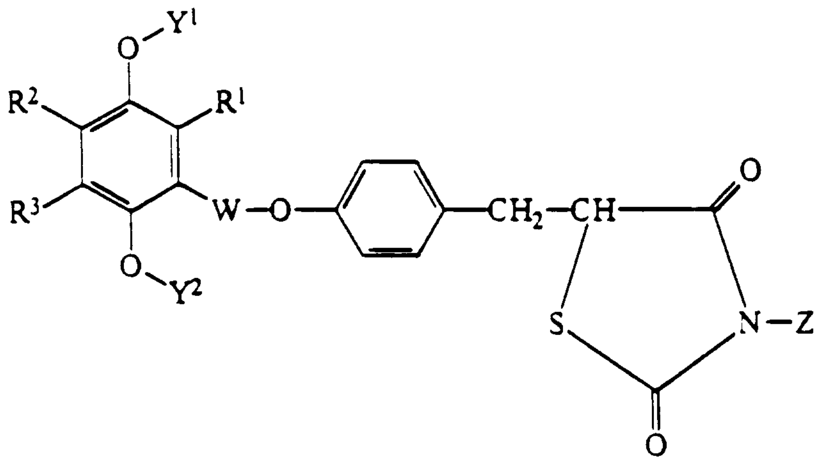

(I-1)

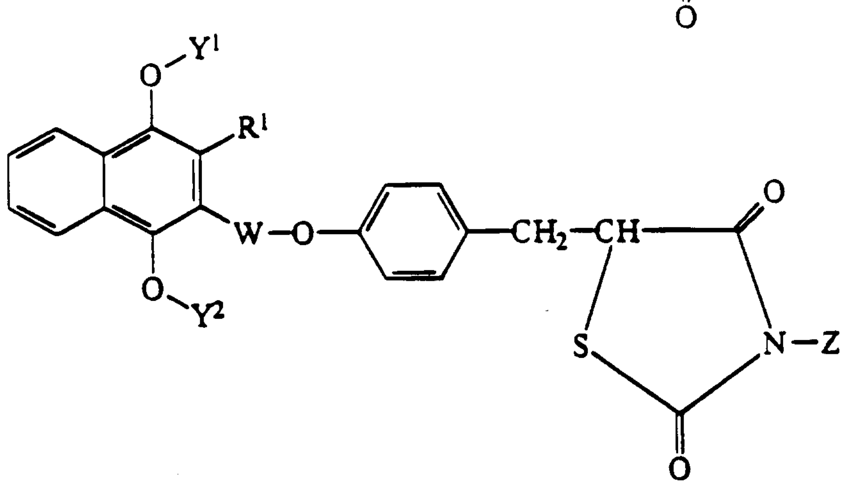

(I-2)

Table 1

| Cpd. No. | $R^1$ | $R^2$ | $R^3$ | $Y^1$ | $Y^2$ | W | Z |
|---|---|---|---|---|---|---|---|
| 1-1 | Me | Me | Me | H | H | single bond | H |
| 1-2 | Me | Me | Me | H | H | single bond | Na |
| 1-3 | Me | Me | Me | H | H | $-CH_2-$ | H |
| 1-4 | Me | Me | Me | H | H | $-CH_2-$ | Na |
| 1-5 | Me | Me | M | H | H | $-(CH_2)_2-$ | H |
| 1-6 | Me | Me | Me | H | H | $-(CH_2)_2-$ | Na |
| 1-7 | Me | Me | Me | H | H | $-(CH_2)_3-$ | H |
| 1-8 | Me | Me | Me | H | H | $-(CH_2)_3-$ | Na |
| 1-9 | Me | Me | Me | H | H | $-(CH_2)_4-$ | H |
| 1-10 | Me | Me | Me | H | H | $-(CH_2)_4-$ | Na |
| 1-11 | Me | Me | Me | Me | Me | single bond | H |
| 1-12 | Me | Me | Me | Me | Me | single bond | Na |
| 1-13 | Me | Me | Me | Me | Me | $-CH_2-$ | H |
| 1-14 | Me | Me | Me | Me | Me | $-CH_2-$ | Na |
| 1-15 | Me | Me | Me | Me | Me | $-(CH_2)_2-$ | H |
| 1-16 | Me | Me | Me | Me | Me | $-(CH_2)_2-$ | Na |
| 1-17 | Me | Me | Me | Me | Me | $-(CH_2)_3-$ | H |
| 1-18 | Me | Me | Me | Me | Me | $-(CH_2)_3-$ | Na |
| 1-19 | Me | Me | Me | Me | Me | $-(CH_2)_4-$ | H |
| 1-20 | Me | Me | Me | Me | Me | $-(CH_2)_4-$ | Na |
| 1-21 | Me | Et | Et | Me | Me | $-(CH_2)_2-$ | Na |
| 1-22 | Me | Bu | Bu | Me | Me | $-(CH_2)_3-$ | H |
| 1-23 | Me | Me | Me | Ac | Ac | single bond | H |
| 1-24 | Me | Me | Me | Ac | Ac | single bond | Na |
| 1-25 | Me | Me | Me | Ac | Ac | $-CH_2-$ | H |
| 1-26 | Me | Me | Me | Ac | Ac | $-CH_2-$ | Na |
| 1-27 | Me | Me | Me | Ac | Ac | $-(CH_2)_2-$ | H |
| 1-28 | Me | Me | Me | Ac | Ac | $-(CH_2)_2-$ | Na |

Table 1 (cont.)

| Cpd. No. | $R^1$ | $R^2$ | $R^3$ | $Y^1$ | $Y^2$ | W | Z |
|---|---|---|---|---|---|---|---|
| 1-29 | Me | Me | Me | Ac | Ac | $-(CH_2)_3-$ | H |
| 1-30 | Me | Me | Me | Ac | Ac | $-(CH_2)_3-$ | Na |
| 1-31 | Me | Me | Me | Ac | Ac | $-(CH_2)_4-$ | H |
| 1-32 | Me | Me | Me | Ac | Ac | $-(CH_2)_4-$ | Na |
| 1-33 | Et | Et | Et | Ac | Ac | $-(CH_2)_2-$ | H |
| 1-34 | iPr | iPr | iPr | Byr | Byr | $-(CH_2)_3-$ | H |
| 1-35 | Me | MeO | MeO | H | H | single bond | H |
| 1-36 | Me | MeO | MeO | H | H | $-CH_2-$ | H |
| 1-37 | Me | MeO | MeO | H | H | $-(CH_2)_2-$ | Na |
| 1-38 | Me | MeO | MeO | H | H | $-(CH_2)_3-$ | H |
| 1-39 | Me | MeO | MeO | H | H | $-(CH_2)_3-$ | Na |
| 1-40 | Me | MeO | MeO | H | H | $-(CH_2)_4-$ | H |
| 1-41 | Me | MeO | MeO | H | H | $-(CH_2)_4-$ | Na |
| 1-42 | Me | MeO | MeO | Me | Me | single bond | H |
| 1-43 | Me | MeO | MeO | Me | Me | single bond | Na |
| 1-44 | Me | MeO | MeO | Me | Me | $-CH_2-$ | H |
| 1-45 | Me | MeO | MeO | Me | Me | $-CH_2-$ | Na |
| 1-46 | Me | MeO | MeO | Me | Me | $-(CH_2)_2-$ | H |
| 1-47 | Me | MeO | MeO | Me | Me | $-(CH_2)_2-$ | Na |
| 1-48 | Me | MeO | MeO | Me | Me | $-(CH_2)_3-$ | H |
| 1-49 | Me | MeO | MeO | Me | Me | $-(CH_2)_3-$ | Na |
| 1-50 | Me | MeO | MeO | Me | Me | $-(CH_2)_4-$ | H |
| 1-51 | Me | MeO | MeO | Me | Me | $-(CH_2)_4-$ | Na |
| 1-52 | Me | MeO | MeO | Ac | Ac | single bond | H |
| 1-53 | Me | MeO | MeO | Ac | Ac | single bond | Na |
| 1-54 | Me | MeO | MeO | Ac | Ac | $-CH_2-$ | H |
| 1-55 | Me | MeO | MeO | Ac | Ac | $-CH_2-$ | Na |
| 1-56 | Me | MeO | MeO | Ac | Ac | $-(CH_2)_2-$ | H |

Table 1 (cont.)

| Cpd. No. | $R^1$ | $R^2$ | $R^3$ | $Y^1$ | $Y^2$ | W | Z |
|---|---|---|---|---|---|---|---|
| 1-57 | Me | MeO | MeO | Ac | Ac | $-(CH_2)_2-$ | Na |
| 1-58 | Me | MeO | MeO | Ac | Ac | $-(CH_2)_3-$ | H |
| 1-59 | Me | MeO | MeO | Ac | Ac | $-(CH_2)_3-$ | Na |
| 1-60 | Me | MeO | MeO | Ac | Ac | $-(CH_2)_4-$ | H |
| 1-61 | Me | MeO | MeO | Ac | Ac | $-(CH_2)_4-$ | Na |
| 1-62 | Me | Me | Me | Boz | Boz | $-(CH_2)_2-$ | H |
| 1-63 | Me | Me | Me | Boz | Boz | $-(CH_2)_3-$ | H |
| 1-64 | Me | Me | Me | Nic | Nic | $-(CH_2)_2-$ | H |
| 1-65 | Me | Me | Me | Nic | Nic | $-(CH_2)_3-$ | H |
| 1-66 | Me | Me | Me | 4-MeBoz | 4-(Me)Boz | $-(CH_2)_3-$ | H |
| 1-67 | Me | Me | Me | 2-MeOBoz | 2-MeOBoz | $-(CH_2)_2-$ | H |
| 1-68 | iPr | Me | Me | 3-ClBoz | 3-ClBoz | $-(CH_2)_3-$ | H |

Table 2

| Cpd. No. | $R^1$ | $Y^1$ | $Y^2$ | W | Z |
|---|---|---|---|---|---|
| 2-1 | H | H | H | $-CH_2-$ | H |
| 2-2 | Me | H | H | $-CH_2-$ | H |
| 2-3 | Cl | H | H | $-CH_2-$ | H |
| 2-4 | H | Me | Me | $-CH_2-$ | H |
| 2-5 | H | Me | Me | $-CH_2-$ | Na |
| 2-6 | Cl | Me | Me | single bond | H |
| 2-7 | Cl | Ac | Ac | single bond | H |
| 2-8 | Cl | Ac | Ac | $-CH_2-$ | H |
| 2-9 | Cl | Ac | Ac | $-(CH_2)_2-$ | H |
| 2-10 | Cl | Ac | Ac | $-(CH_2)_3-$ | H |
| 2-11 | Cl | Ac | Ac | $-CH_2-C(CH_3)_2-CH_2-$ | H |
| 2-12 | Me | Me | Me | $-CH_2-$ | H |
| 2-13 | Me | Me | Me | $-CH_2-$ | Na |
| 2-14 | H | Me | Me | $-(CH_2)_2-$ | H |
| 2-15 | H | Me | Me | $-(CH_2)_2-$ | Na |
| 2-16 | H | Me | Me | $-(CH_2)_3-$ | H |
| 2-17 | H | Me | Me | $-(CH_2)_3-$ | Na |
| 2-18 | H | Me | Me | $-(CH_2)_4-$ | H |
| 2-19 | H | Me | Me | $-(CH_2)_4-$ | Na |
| 2-20 | H | Me | Me | $-(CH_2)_5-$ | H |
| 2-21 | H | Me | Me | $-CH_2-C(CH_3)_2-CH_2-$ | H |
| 2-22 | H | Boz | Boz | $-(CH_2)_2-$ | H |
| 2-23 | H | Boz | Boz | $-(CH_2)_3-$ | H |
| 2-24 | H | Nic | Nic | $-(CH_2)_2-$ | H |
| 2-25 | H | Nic | Nic | $-(CH_2)_3-$ | H |

Of the compounds listed above, preferred compounds are Compounds Nos.:

1-7. 5-{4-[3-(2,5-Dihydroxy-3,4,6-trimethylphenyl)-propoxy]benzyl}thiazolidine-2,4-dione;
1-14. 5-[4-(2,5-Dimethoxy-3,4,6-trimethylbenzyloxy)-benzyl]thiazolidine-2,4-dione sodium salt;
1-17. 5-{4-[3-(2,5-Dimethoxy-3,4,6-trimethylphenyl)-propoxy]benzyl}thiazolidine-2,4-dione;
1-18. 5-{4-[3-(2,5-Dimethoxy-3,4,6-trimethylphenyl)-propoxy]benzyl}thiazolidine-2,4-dione sodium salt;
1-20. 5-{4-[4-(2,5-Dimethoxy-3,4,6-trimethylphenyl)-butoxy]benzyl}thiazolidine-2,4-dione sodium salt;
1-23. 5-[4-(2,5-Diacetoxy-3,4,6-trimethylphenoxy)-benzyl]thiazolidine-2,4-dione;
1-27. 5-{4-[2-(2,5-Diacetoxy-3,4,6-trimethylphenyl)-ethoxy]benzyl}thiazolidine-2,4-dione;
1-30. 5-{4-[3-(2,5-Diacetoxy-3,4,6-trimethylphenyl)-propoxy]benzyl}thiazolidine-2,4-dione sodium salt;
1-47. 5-{4-[2-(2,3,4,5-Tetramethoxy-6-methylphenyl)-ethoxy]benzyl}thiazolidine-2,4-dione sodium salt;
1-49. 5-{4-[3-(2,3,4,5-Tetramethoxy-6-methylphenyl)-propoxy]benzyl}thiazolidine-2,4-dione sodium salt;
1-50. 5-{4-[4-(2,3,4,5-Tetramethoxy-6-methylphenyl)-butoxy]benzyl}thiazolidine-2,4-dione;
1-51. 5-{4-[4-(2,3,4,5-Tetramethoxy-6-methylphenyl)-butoxy]benzyl}thiazolidine-2,4-dione sodium salt;
2-4. 5-[4-(2,7-Dimethoxynaphthylmethoxy)benzyl]-thiazolidine-2,4-dione;
2-5. 5-[4-(2,7-Dimethoxynaphthylmethoxy)benzyl]-thiazolidine-2,4-dione sodium salt;
2-12. 5-[4-(2,7-Dimethoxy-8-methylnaphthylmethoxy)-benzyl]thiazolidine-2,4-dione;
2-14. 5-{4-[2-(2,7-Dimethoxynaphthyl)ethoxy]-benzyl}thiazolidine-2,4-dione; and
2-15. 5-{4-[2-(2,7-Dimethoxynaphthyl)ethoxy]-benzyl}thiazolidine-2,4-dione sodium salt.

Of these, we more prefer Compounds Nos. 1-18, 1-27, 2-4 and 2-14, and the most preferred compounds are Compounds Nos. 1-18 and 2-14.

The compounds of the present invention may be prepared by a variety of processes well known in the art for the preparation of this type of compound. For example, they may be prepared as illustrated by the following Methods A to H.

Method A:

Method A consists of the procedure described in European Patent Publication No. 139 421 (Japanese Patent Kokai Application No. Sho 60-51189 (=Japanese Patent Publication No. Hei 2-31079). The desired

compound of formula (I) can be prepared by reacting a compound of general formula (II):

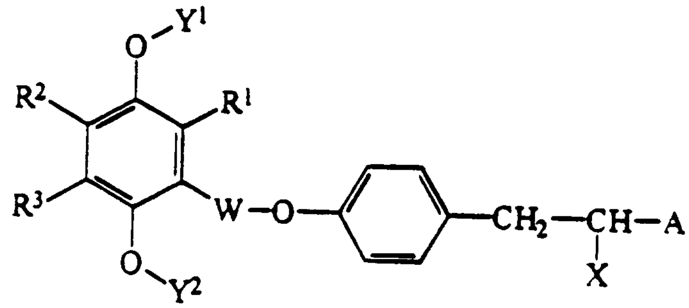

(II)

(in which $R^1$, $R^2$, $R^3$, $Y^1$, $Y^2$ and W are as defined above; A represents a carboxyl, alkoxycarbonyl or carbamoyl group, or a group of formula -COOM, where M represents a cation, especially a metal atom; and X represents a halogen atom), which may be prepared as described in the above cited patent, in relation to the α-halocarboxylic acids used as starting materials and/or in the "Referential Examples", with thiourea to produce an intermediate of formula (III):

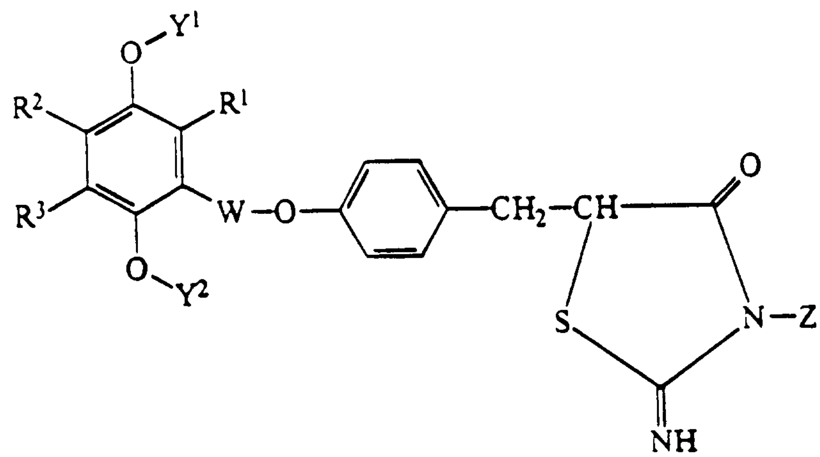

(III)

(in which $R^1$, $R^2$, $R^3$, $Y^1$, $Y^2$ and W are as defined above) and then hydrolysing the compound of formula (III), for example as described in the cited patent.

Examples of the alkoxycarbonyl groups which may be represented by A include the methoxycarbonyl, ethoxycarbonyl, isopropoxycarbonyl and butoxycarbonyl groups. In the group of formula -COOM, M represents a metal atom, for example a sodium, potassium, calcium or aluminium, or an equivalent cation, such as an ammonium ion. X represents a halogen atom, such as a chlorine, bromine or iodine atom.

The reaction of the compound of formula (II) with thiourea is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol, ethanol, propanol, butanol or ethylene glycol monomethyl ether; ethers, such as tetrahydrofuran or dioxane; ketones, such as acetone; sulphoxides, such as dimethyl sulphoxide or sulpholane; and amides, especially fatty acid amides, such as dimethylformamide or dimethylacetamide. There is no particular limitation upon the molar ratio of the compound of formula (II) to the thiourea used, but the reaction is preferably carried out using at least a slight molar excess of the thiourea per mole of the compound of formula (II). It is more preferred to use from 1 to 2 moles of thiourea per mole of the compound of formula (II).

The reaction can take place over a wide range of temperatures, and the precise reaction temperature is not critical to the invention, and the preferred temperature may vary depending upon the nature of the starting material and the solvent used, . In general, we find it convenient to carry out the reaction at the boiling point of the solvent or at a temperature of from 80 to 150 °C. The time required for the reaction may

also vary widely, depending on many factors, notably the reaction temperature and the nature of the reagents and solvent employed. However, provided that the reaction is effected under the preferred conditions outlined above, a period of from 1 to several tens of hours will usually suffice.

After that, the compound of formula (III) may be hydrolyzed by heating it in an appropriate solvent in the presence of water and of an organic acid, such as acetic acid, or a mineral acid, such as sulphuric acid or hydrochloric acid. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: sulphoxides, such as sulpholane; and alcohols, such as methanol, ethanol and ethylene glycol monomethyl ether. The amount of the acid to be used is normally and preferably from 0.1 to 10 moles, more preferably from 0.2 to 3 moles, per mole of the compound of formula (III). Water or an aqueous solvent is normally added in a large excess relative to the molar amount of the compound of formula (III).

The reaction can take place over a wide range of temperatures, and the precise reaction temperature is not critical to the invention. In general, we find it convenient to carry out the reaction at a temperature of from 50 to 100 °C. The time required for the reaction may also vary widely, depending on many factors, notably the reaction temperature and the nature of the reagents and solvent employed. However, provided that the reaction is effected under the preferred conditions outlined above, a period of from several hours to several tens of hours will usually suffice.

After the hydrolysis, $Y^1$ and $Y^2$ in the compound of formula (I) are each usually a hydrogen atom or the corresponding alkyl group. Where $Y^1$ and $Y^2$ are each acyl groups, they may remain intact, depending upon the choice of reaction conditions.

Method B:

Method B involves the preparation of a compound of formula (I) by the procedure described in J. Med. Chem., 1538 (1991).

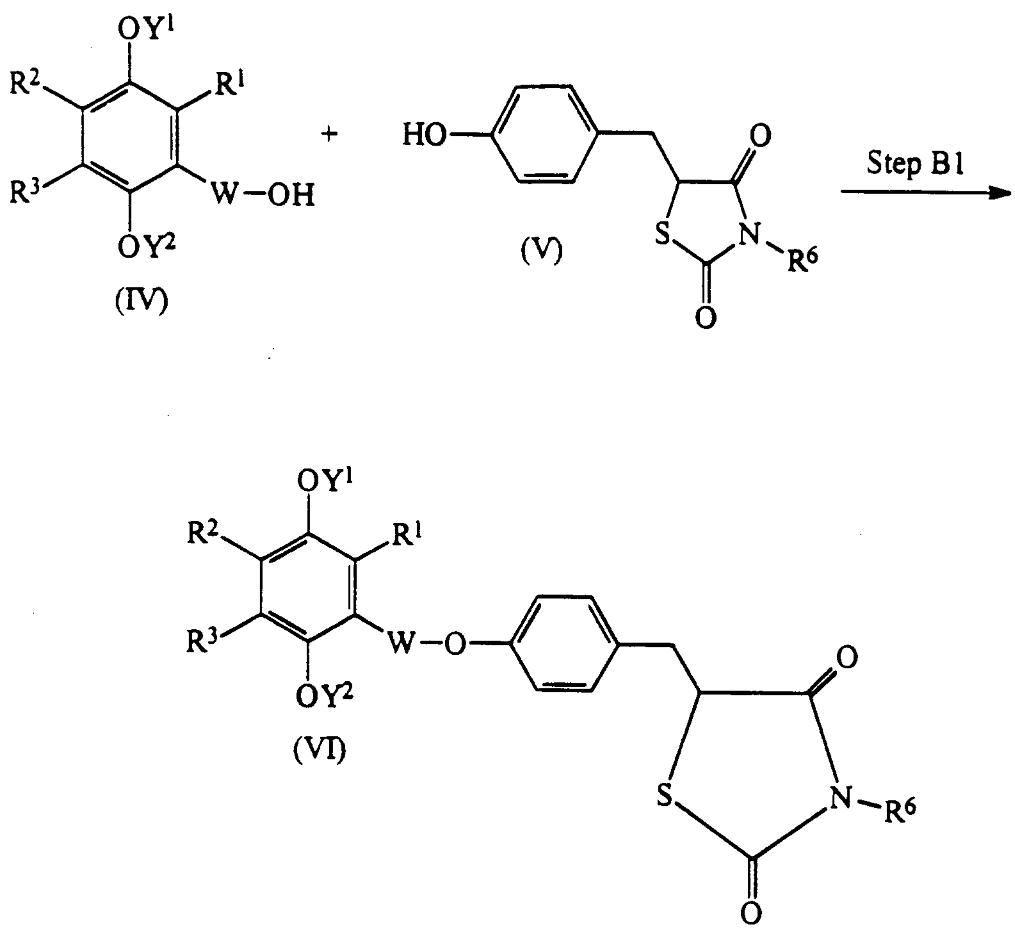


(IV) (V) (VI)

In the above formulae, $R^1$, $R^2$, $R^3$, $Y^1$, $Y^2$ and W are as defined above and $R^6$ represents a hydrogen atom or a protecting group.

In Method B, the alcohol compound of formula (IV), which is used as a starting material, can be prepared by the procedure described in, for example, J. Am. Chem. Soc., 64, 440 (1942), J. Am. Chem. Soc., 94, 227 (1972), J. Chem. Soc. Perkin Trans. I., 1591 (1983), Japanese Patent Kokai Application No. Sho 58-83698 (= Japanese Patent Publication Ho. Hei 1-33114), Japanese Patent Kokai Application No. 58-174342 (= Japanese Patent Publication No. Hei 1-39411) or J. Takeda Res. Lab., 45, No. 3 & 4, 73 (1986) followed by conventional conversion reactions. The desired compound of formula (VI) can then be prepared by a dehydration condensation reaction, for example that known as the Mitsunobu reaction (Fieser & Fieser, "Reagents for Organic Synthesis", Vol. 6, pp 645, A Wiley-Interscience Publication, edited by John Wiley & Sons), between the compound of formula (IV) and an optionally protected thiazolidine compound of formula (V).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene or toluene; aliphatic hydrocarbons, such as hexane or heptane; ethers, such as tetrahydrofuran or dioxane; halogenated hydrocarbons, especially halogenated aliphatic hydrocarbons, such as methylene chloride; and sulphoxides, such as dimethyl sulphoxide. The molar ratio of the compound of formula (IV) to the compound of formula (V) is not particularly critical but it is preferred to use from 1 to 3 moles of the compound of formula (V) per mole of the compound of formula (IV).

The reaction can take place over a wide range of temperatures, and the precise reaction temperature is not critical to the invention. In general, we find it convenient to carry out the reaction at a temperature of from -20 to 150 °C. The time required for the reaction may also vary widely, depending on many factors, notably the reaction temperature and the nature of the reagents and solvent employed. However, provided that the reaction is effected under the preferred conditions outlined above, a period of from 10 minutes to several tens of hours will usually suffice.

Where the compound of formula (VI) thus obtained has a protecting group, for example a trityl group, deprotection may, if desired, be achieved by treating the compound of formula (VI) with an organic acid, such as trifluoroacetic acid, to give a compound of formula (I). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: ethers, such as tetrahydrofuran or dioxane; and halogenated hydrocarbons, especially halogenated aliphatic hydrocarbons, such as methylene chloride. The molar ratio of trifluoroacetic acid to the compound of formula (VI) is preferably from 0.5 : 1 to a large excess.

The reaction can take place over a wide range of temperatures, and the precise reaction temperature is not critical to the invention, and the preferred temperature may vary depending upon the nature of the starting material and the solvent used. In general, we find it convenient to carry out the reaction at a temperature of from -20 to 40 °C. The time required for the reaction may also vary widely, depending on many factors, notably the reaction temperature and the nature of the reagents and solvent employed. However, provided that the reaction is effected under the preferred conditions outlined above, a period of from several minutes to several tens of hours will usually suffice.

Method C:

In Method C, the desired compound of formula (I) can be prepared by converting a compound of formula (IV), described in Method B, to an active ester derivative or to a halogenated compound and reacting the product with a compound of formula (V).

In a first step, the compound of formula (IV) is converted to an active ester compound, such as a methanesulphonate, benzenesulphonate or toluenesulphonate, by conventional means, or to a halogenated compound, such as the chloride, bromide or iodide, by conventional means. The desired compound of formula (I) can then be prepared by reacting the active ester compound or halogenated compound thus obtained with the compound of formula (V).

The reaction of the active ester compound or halogenated compound with the compound of formula (V) is normally and preferably carried out in the presence of a base, for example, an inorganic base, such as an alkali metal carbonate (for example sodium carbonate or potassium carbonate), or an alkali metal hydroxide (for example sodium hydroxide or potassium hydroxide); an alkali metal alcoholate, such as sodium

methoxide, sodium ethoxide or potassium t-butoxide; or a metal hydride, such as sodium hydride, potassium hydride or lithium hydride. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. The preferred solvent to be used will vary depending upon the nature of the base used. However, examples of suitable solvents include: aromatic hydrocarbons, such as benzene, toluene or xylene; ethers, such as diethyl ether, tetrahydrofuran or dioxane; amides, especially fatty acid amides, such as dimethylformamide or dimethylacetamide; and organic sulphur compounds, such as dimethyl sulphoxide or sulpholane. Of these, we prefer the amides. The molar ratio of the compound of formula (V) to the base is normally from 0.5 : 1 to 5 : 1, more preferably from 1 : 1 to 3 : 1. The molar ratio of the compound of formula (V) to the active ester compound or the halogenated compound is normally from 0.5 : 1 to 4 : 1, more preferably from 1 : 1 to 3 : 1.

The reaction can take place over a wide range of temperatures, and the precise reaction temperature is not critical to the invention, and the preferred temperature to be used will vary depending upon the nature of the starting material, the base and solvent used. In general, we find it convenient to carry out the reaction at a temperature of from 0 to 50°C, more preferably from 5 to 20°C. The time required for the reaction may also vary widely, depending on many factors, notably the reaction temperature and the nature of the reagents and solvent employed. However, provided that the reaction is effected under the preferred conditions outlined above, a period of from several minutes to several tens of hours will usually suffice.

The protecting group can then, if desired, be eliminated by the procedure described in Method B.

Method E:

In this method, a compound of formula (I), in which $Y^1$ and $Y^2$ both represent hydrogen atoms, can be prepared by Reaction Scheme E summarized below:

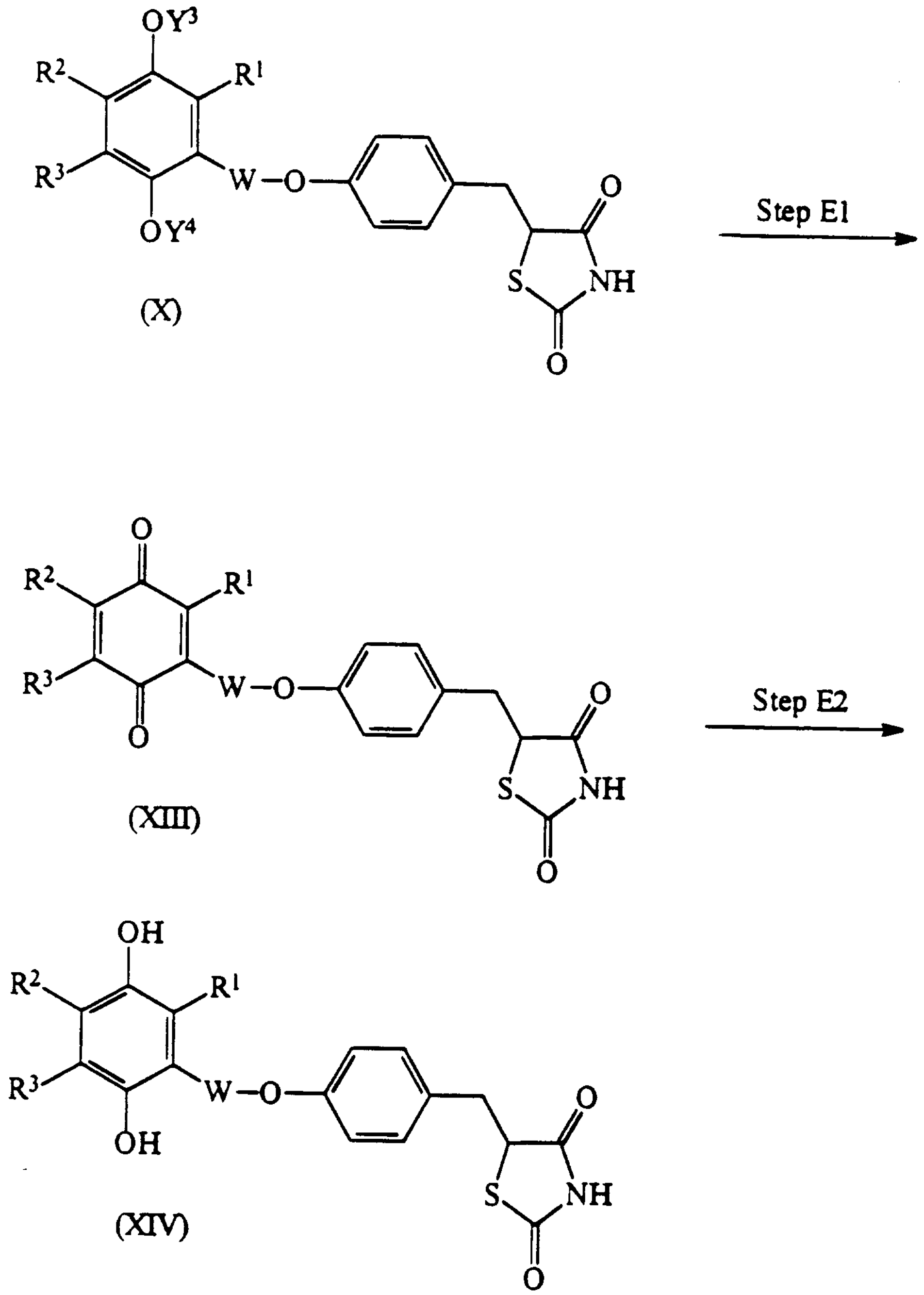


(X)

(XIII)

(XIV)

In the above formulae, $R^1$, $R^2$, $R^3$, $Y^3$, $Y^4$ and W are as defined above.

In step E1 of this reaction scheme, a compound of formula (X), in which $Y^3$ and $Y^4$ each represents a lower alkyl group, particularly a methyl group, is converted by oxidation using ceric ammonium nitrate to a compound of formula (XIII) by the procedure described in Fieser & Fieser, "Reagents for Organic Synthesis", Vol. 7, pp. 55, A Wiley-Interscience Publication, edited by John Wiley & Sons. The oxidation reaction using cerium ammonium nitrate is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; nitriles, such as acetonitrile; ketones, such as acetone; and mixtures of any two or more of the above solvents. The amount of cerium ammonium nitrate used is not particularly critical but it is preferred to use from 1 to 10 moles of cerium ammonium nitrate per mole of the compound of formula (X). The reaction can take place over a wide range of temperatures, and the precise reaction temperature is not critical to the invention, although the preferred temperature may vary

depending upon the nature of the starting material and the solvent used. In general, we find it convenient to carry out the reaction at a temperature of from -10 to 40 °C. The time required for the reaction may also vary widely, depending on many factors, notably the reaction temperature and the nature of the reagents and solvent employed. However, provided that the reaction is effected under the preferred conditions outlined above, a period of from several minutes to several tens of hours will usually suffice.

Subsequently, the compound of formula (XIV) can be prepared from the compound of formula (XIII) by reduction, for example, catalytic reduction, or using a reducing agent, such as a hydride (for example sodium borohydride) or a metal (for example, zinc or iron).

If desired, the starting compound can be subjected first to oxidation using cerium ammonium nitrate, for example as shown in Reaction Scheme E':

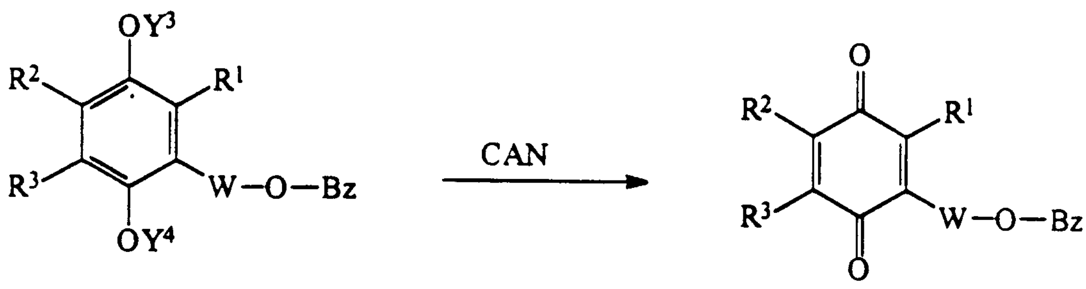


(XV) (XVI)

(in which $R^1$, $R^2$, $R^3$, $Y^3$, $Y^4$ and W are as defined above and Bz represents a benzyl or substituted benzyl group). As an example, this method is applicable to a compound of formula (XV), that is, a compound of formula (IV) in which the hydroxyl group is protected by a benzyl group, to produce a compound of formula (XVI).

Method F:

This method consists in the preparation of a compound of formula (I), in which $Y^1$ and $Y^2$ each represents an acyl group.

In this, a quinone or naphthoquinone compound, for example the compound of formula (XIV), which may have been prepared as described in Method E, and after isolation from the reaction mixture or without isolation, is subjected to acylation by conventional means to give a compound equivalent to the compound of formula (X) but in which the alkyl groups represented by $Y^3$ and $Y^4$ are replaced by acyl groups.

This reaction can, if desired, be conducted in the step preparing the starting materials. For example, as shown in Reaction Scheme F:

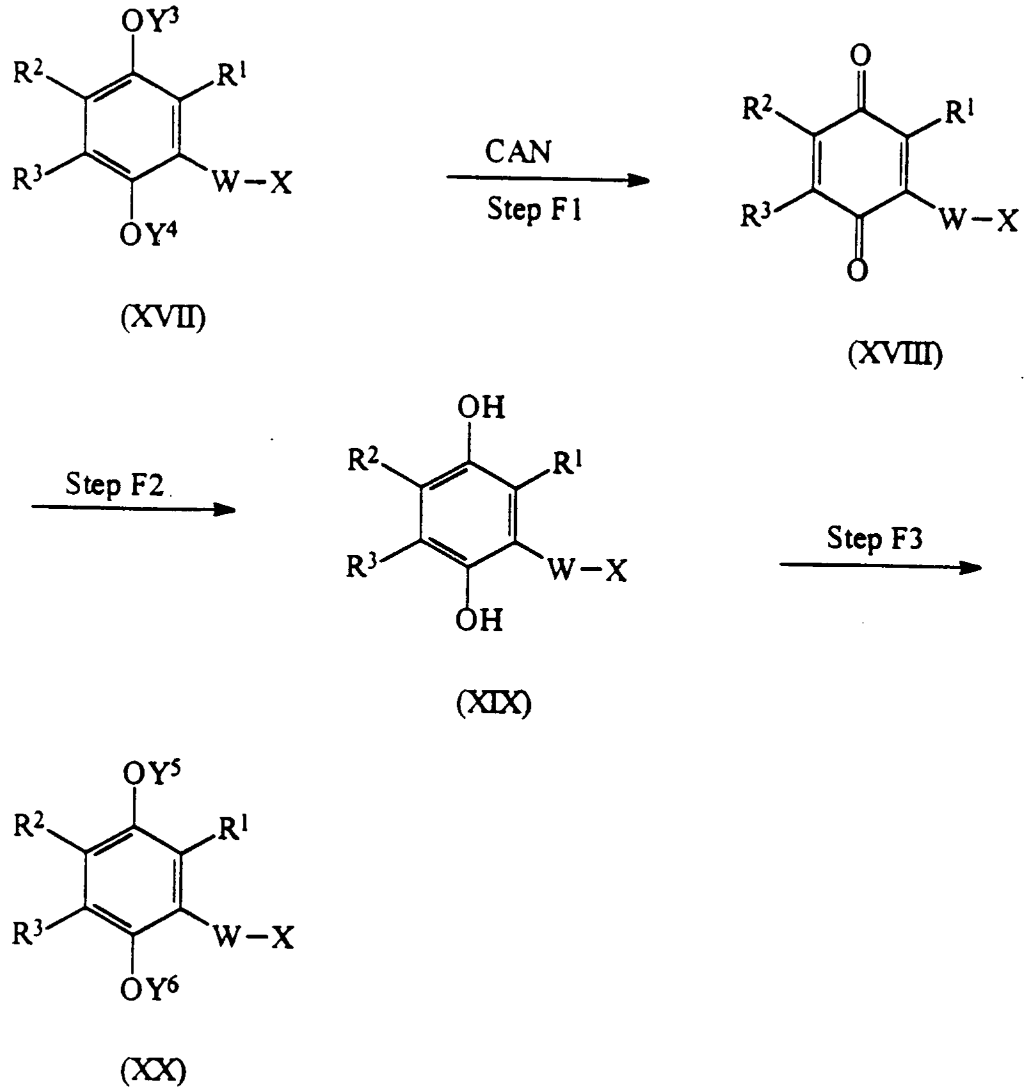


(XVII) (XVIII) (XIX) (XX)

In the above formulae, $R^1$, $R^2$, $R^3$, $Y^3$, $Y^4$ and W are as defined above; X represents a halogen atom, such as a chlorine, bromine or iodine atom; and $Y^5$ and $Y^6$ are the same or different, preferably the same, and each represents an acyl group within the definition of $Y^1$ and $Y^2$.

In this reaction scheme, a compound of formula (XVII), in which $Y^3$ and $Y^4$ each represents a lower alkyl group (particularly a methyl group), is treated by the process described in Method E to produce a compound of formula (XVIII). Subsequently, a diacyl compound of formula (XX) can be obtained for use as a starting material by reducing the compound of formula (XVIII) by the procedure described in Method E to give a compound of formula (XIX), and then acylating the product, to give the compound of formula (XX).

Acylation can be carried out after isolation or without isolation of the compound of formula (XIX). Where acylation is conducted without isolation of the compound of formula (XIX), the compound of formula (XX) can be obtained by reducing the compound of formula (XVIII) using a metal, such as zinc or iron, in the presence of an acylating agent, such as an acid anhydride (for example acetic anhydride) or a halogenated acyl compound (for example acetyl chloride). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: organic acids, such as acetic acid or propionic acid; and organic bases, such as pyridine.

Method G:

In Reaction Scheme G, the desired compound of formula (I), for example in which Z represents a sodium atom, can be prepared in the form of a salt, that is by replacing the hydrogen atom of the imide group with a metal atom by reacting a compound of formula (I) in which Z represents a hydrogen atom with a suitable base by conventional means. There is no particular limitation upon the nature of the base used. Examples of such bases include: sodium hydroxide, alcoholates, such as sodium methoxide or sodium ethoxide, and sodium salts of organic acids, such as sodium 2-ethylhexanoate. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. The preferred solvent used may vary, depending upon the nature of the base used, but examples of the solvents which may be used include: lower alcohols, such as methanol or ethanol; esters, such as ethyl acetate or propyl acetate; ethers, such as tetrahydrofuran or dioxane; water; and mixtures of any two or more of the above solvents. Salts of other metals, for example potassium or calcium, or the corresponding salts of basic amino acids or other organic bases can be prepared in a similar manner to the preparation of the sodium salts described above.

Method H:

This method can be applied to the preparation of a compound of formula (I), in which $R^2$ and $R^3$ together form a benzene ring which is unsubstituted or is substituted by from 1 to 4 of substituents A, defined and exemplified above; and W is a single bond, as shown in Reaction Scheme H:

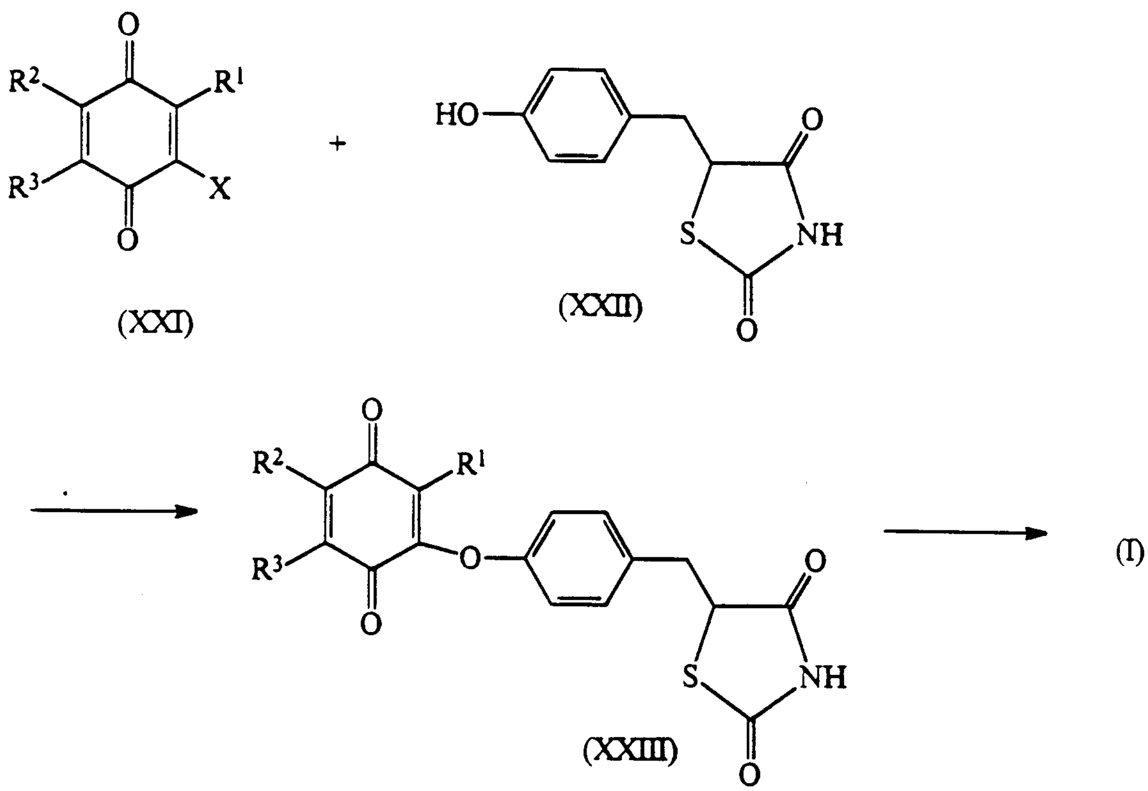

(XXI) (XXII) (XXIII) (I)

(in which $R^1$, $R^2$, $R^3$ and X are as defined above)

The reaction is normally and preferably carried out in the presence of a base or using an alkali metal salt (for example the sodium salt) of 5-(4-hydroxybenzyl)-thiazolidine-2,4-dioneof formula (XXII). The base used, the solvent used, the reaction temperature and the time required for the reaction are similar to those of Method C.

Alternatively, a compound of formula (XXI) may be reacted with 4-hydroxynitrobenzene or with a salt thereof, to give a 3-halo-2-(4-nitrophenoxy)-1,4-naphthoquinone derivative and then the product is converted to the compound of formula (II) by the procedure of the literature described in Method A. Subsequently,

following the procedure of Method A, the compound of formula (I) can be prepared from the compound of formula (II). The reaction is carried out under the same conditions as those described in Method A.

After completion of any of the above reactions, the desired compounds can be recovered from the reaction mixture and, if necessary, purified by conventional means, for example by the various chromatography techniques, such as column chromatography, or by recrystallization, reprecipitation or the like. An example of such a recovery procedure comprises: adding a solvent to the reaction mixture and then distilling off the solvent from the extract. The residue thus obtained can be purified by column chromatography through silica gel or the like to give the desired compound in a pure state. In the case of compounds used as intermediates in the above reactions, they may be recovered, for example as illustrated above, or they may, in many cases, be used without intermediate purification.

Moreover, where the compound obtained comprises a mixture of various isomers, these isomers can be separated by conventional separating means in an appropriate stage.

## BIOLOGICAL ACTIVITY

The thiazolidine compounds of the present invention showed excellent hypoglycemic activity and an outstanding inhibitory action against hepatic gluconeogenesis in a test system using genetically diabetic animals. Accordingly, it is expected that the compounds of the invention will be useful for the treatment and/or prevention of diabetes, diabetic complications, hyperlipidemia, hyperlipoperoxidemia, obesity-related hypertension, osteoporosis and the like.

The compounds of the present invention can be administered in various forms, depending on the disorder to be treated and the condition of the patient, as is well known in the art. For example, where the compounds are to be administered orally, they may be formulated as tablets, capsules, granules, powders or syrups; or for parenteral administration, they may be formulated as injections (intravenous, intramuscular or subcutaneous), drop infusion preparations or suppositories. For application by the ophthalmic mucous membrane route, they may be formulated as eyedrops or eye ointments. These formulations can be prepared by conventional means, and, if desired, the active ingredient may be mixed with any conventional additive, such as a vehicle, a binder, a disintegrator, a lubricant, a corrigent, a solubilizing agent, a suspension aid, an emulsifying agent or a coating agent. Although the dosage will vary depending on the symptoms, age and body weight of the patient, the nature and severity of the disorder to be treated or prevented, the route of administration and the form of the drug, for the treatment of diabetes, diabetic complications and/or hyperlipemia, a daily dosage of from 1 to 1000 mg of the compound is recommended for an adult human patient, and this may be administered in a single dose or in divided doses.

The activity of the compounds of the present invention is illustrated by the following Experiment.

## Experiment

### Hypoglycemic activity

The test animals used were diabetic male mice of the KK strain, each having a body weight more than 40 g. Each animal was orally administered 50 mg/kg of a test compound and then allowed to feed freely for 18 hours. At the end of this time, blood was collected from the tail veins without anesthesia. The blood glucose level (BGL) was determined by means of a glucose analyzer (GL-101, manufactured by Mitsubishi Kasei Co.).

The blood glucose lowering rate was calculated by the following equation:

$$\text{Blood glucose lowering rate (\%)} = [(BGL_s - BGL_t)/BGL_s] \times 100$$

where:

$BGL_s$ is the BGL in the group administered a solvent; and

$BGL_t$ is the BGL in the group administered a test compound.

The results are shown in the following Table, in which each compound of the present invention is identified by the number of one of the following Examples in which its preparation is illustrated.

As a control, we also used the following prior art compounds as test compounds:

5-{4-[2-Methyl-2-hydroxy-4-(3,5,6-trimethyl-1,4-benzoquinon-2-yl)butoxy]benzyl}thiazolidine-2,4-dione (which is the compound of Example 1 described in European Patent Publication No. 441 605). This is identified as "Control 1"; and

5-{4-[4-(2,5-Dihydroxy-3,4,6-trimethylphenyl)-2-hydroxy-2-methylbutoxy]benzyl}thiazolidine-2,4-dione (which

is the compound of Example 3 described in European Patent Publication No. 441 605). This is identified as "Control 2".

Table

| Compound | BGL lowering rate (%) |
|---|---|
| Compound of Example 7 | 24.0 |
| Compound of Example 9 | 28.8 |
| Compound of Example 10 | 46.0 |
| Compound of Example 12 | 24.0 |
| Compound of Example 16 | 20.2 |
| Compound of Example 18 | 22.0 |
| Compound of Example 19 | 26.6 |
| Compound of Example 21 | 33.4 |
| Compound of Example 23 | 24.4 |
| Compound of Example 31 | 32.9 |
| Compound of Example 33 | 28.4 |
| Compound of Example 34 | 40.0 |
| Control 1 | -0.5 |
| Control 2 | 10.4 |

As can be seen from the results shown in the Table, the compounds of the present invention showed a much greater activity than did either of the compounds of the prior art.

The preparation of the compounds of the present invention is further illustrated by the following non-limiting Examples, and the preparation of various intermediates used in these Examples is illustrated in the subsequent Preparations.

## EXAMPLE 1

### 5-[4-(2,4,5-Trimethyl-3,6-dimethoxyphenoxy)benzyl]-thiazolidine-2,4-dione (Compound No. 1-11)

A mixture of 5.7 g of butyl 2-bromo-3-[4-(2,4,5-trimethyl-3,6-dimethoxyphenoxy)phenyl]propionate (prepared as described in Preparation 1), 1.2 g of thiourea and 10 ml of sulpholane was heated at 120°C for 5 hours under an atmosphere of nitrogen, and then 20 ml of ethylene glycol monomethyl ether and 10 ml of 2 N aqueous hydrochloric acid were added to the resulting mixture. The mixture was then heated at 100°C for 5 hours, after which the reaction mixture was poured into water and then extracted with benzene. The extract was washed with water and dried over anhydrous sodium sulphate. The solvent was removed from the extract by distillation under reduced pressure, and the residue thus obtained was purified by column chromatography through silica gel, using a 9 : 1 by volume mixture of benzene and ethyl acetate as the eluent, to give 4.7 g of the title compound as a white glassy powder softening at 47 - 50°C.

Nuclear Magnetic Resonance Spectrum (hexadeuterated dimethyl sulphoxide) δ ppm:
1.97 (3H, singlet);
2.11 (3H, singlet);
2.15 (3H, singlet);
3.04 (1H, doublet of doublets, J = 9 & 14 Hz);
3.32 (1H, doublet of doublets, J = 4 & 14 Hz);
3.54 (3H, singlet);
3.61 (3H, singlet);
4.85 (1H, doublet of doublets, J = 4 & 9 Hz);
6.70 (2H, doublet, J = 8 Hz);
7.15 (2H, doublet, J = 8 Hz).

EXAMPLE 2

5-{4-[2-(2,4,5-Trimethyl-3,6-dimethoxyphenyl)ethoxy]-benzyl}thiazolidine-2,4-dione (Compound No. 1-15)

3.2 g of diethyl azodicarboxylate were added dropwise, whilst ice-cooling and under an atmosphere of nitrogen, to a solution of 3.5 g of 2-(2,4,5-trimethyl-3,6-dimethoxyphenyl)ethanol, 7.3 g of 5-(4-hydroxybenzyl)-3-triphenylmethylthiazolidine-2,4-dione (prepared as described in Preparation 23) and 4.9 g of triphenylphosphine in 100 ml of tetrahydrofuran, and the resulting mixture was stirred at room temperature for 5 hours. At the end of this time, the reaction mixture was poured into water, after which it was extracted with ethyl acetate. The extract was washed with a saturated aqueous solution of sodium chloride and dried over anhydrous sodium sulphate. The solvent was then removed from the extract by distillation under reduced pressure, and the resulting residue was purified by column chromatography through silica gel, using a 4 : 1 by volume mixture of hexane and ethyl acetate as the eluent, to give 5-{4-[2-(2,4,5-trimethyl-3,6-dimethoxyphenyl)ethoxy]benzyl}-3-triphenylmethylthiazolidine-2,4-dione as an oily intermediate. 50 ml of trifluoroacetic acid were added, whilst ice-cooling, to 7.9 g of the intermediate, and the resulting mixture was stirred for 1 hour. At the end of this time, the reaction mixture was diluted with water, after which it was extracted with ethyl acetate. The extract was washed twice, each time with a saturated aqueous solution of sodium hydrogencarbonate; it was then dried over anhydrous sodium sulphate. The solvent was then removed by distillation under reduced pressure, and the residue thus obtained was purified by column chromatography through silica gel, using a 3 : 1 by volume mixture of hexane and ethyl acetate as the eluent, to give 3.6 g of the title compound softening at 44 - 45 °C.

EXAMPLE 3

5-{4-[3-(2,5-Dimethoxy-3,4,6-trimethylphenyl)-propoxy]benzyl}thiazolidine-2,4-dione (Compound No. 1-17)

8.01 g of 5-(4-hydroxybenzyl)thiazolidine-2,4-dione were added in small amounts, whilst ice-cooling, to a suspension prepared by adding 80 ml of dimethylformamide to 3.45 g of sodium hydride (as a 55% w/w dispersion in mineral oil, and which had previously been washed twice with dry hexane). The resulting mixture was stirred at the same temperature for 30 minutes, after which a solution of 13.73 g of 3-(2,5-dimethoxy-3,4,6-trimethylphenyl)propyl iodide (prepared as described in Preparation 15), in 20 ml of dimethylformamide was added dropwise to the solution. The mixture was then stirred at room temperature for 1.5 hours. At the end of this time, the reaction mixture was poured into 300 ml of ice-water, after which it was extracted with ethyl acetate. The extract was washed twice, each time with a saturated aqueous solution of sodium chloride, and dried over anhydrous sodium sulphate. The solvent was then removed from the extract by distillation under reduced pressure, and the residue thus obtained was purified by column chromatography through silica gel, using a gradient elution method with mixtures of hexane and ethyl acetate ranging from 3 : 1 to 2 : 1 by volume as the eluent, to give 6.7 g of the title compound, melting at 111 - 113 °C.

EXAMPLE 4

5-[4-(2,5-Dihydroxy-3,4,6-trimethylphenoxy)benzyl]-thiazolidine-2,4-dione (Compound No. 1-1)

50 mg of sodium borohydride were added, whilst ice-cooling, to a mixture of 480 mg of 5-[4-(3,5,6-trimethyl-1,4-benzoquinon-2-yloxy)benzyl]thiazolidine-2,4-dione (prepared as described in Preparation 2) in 8 ml of ethanol, and the resulting mixture was stirred at room temperature for 30 minutes. At the end of this time, the reaction mixture was poured into cooled dilute aqueous hydrochloric acid to precipitate crystals, which were collected by filtration, thus giving 470 mg of the title compound, melting at 124 - 130 °C.

EXAMPLE 5

5-[4-(2,4,5-Trimethyl-3,6-dimethoxyphenoxy)benzyl]-thiazolidine-2,4-dione sodium salt (Compound No. 1-12)

35 mg of sodium methoxide were added to a solution of 250 mg of 5-[4-(2,4,5-trimethyl-3,6-dimethoxyphenoxy)benzyl]thiazolidine-2,4-dione (prepared as described in Example 1) in 2 ml of methanol. At the end of this time, the solvent was removed from the reaction mixture by distillation under reduced pressure,

to give 240 mg of the title compound as a colourless glassy powder, melting at 120 - 125°C (softening point).

Nuclear Magnetic Resonance Spectrum (hexadeuterated dimethyl sulphoxide) δ ppm:

1.98 (3H, singlet);
2.11 (3H, singlet);
2.15 (3H, singlet);
2.63 (1H, doublet of doublets, J = 10 & 14 Hz);
3.33 (1H, doublet of doublets, J = 3 & 14 Hz);
3.56 (3H, singlet);
3.61 (3H, singlet);
4.14 (1H, doublet of doublets, J = 3 & 10 Hz);
6.64 (2H, doublet, J = 8 Hz);
7.10 (2H, doublet, J = 8 Hz).

## EXAMPLE 6

5-{4-[2-(2,4,5-Trimethyl-3,6-dimethoxyphenyl)-ethoxy]benzyl}thiazolidine-2,4-dione sodium salt (Compound No. 1-16)

0.12 g of sodium 2-ethylhexanoate was added to a solution of 0.3 g of 5-{4-[2-(2,4,5-trimethyl-3,6-dimethoxyphenyl)ethoxy]benzyl}thiazolidine-2,4-dione (prepared as described in Example 2) in 10 ml of ethyl acetate, and the resulting mixture was stirred at room temperature for 17 hours. At the end of this time, the solvent was removed from the reaction mixture by distillation under reduced pressure. The resulting crystalline residue was then washed with 10 ml of hexane, to give 252 mg of the title compound, melting at 165 - 170°C.

## EXAMPLE 7

5-[4-(2,5-Diacetoxy-3,4,6-trimethylphenoxy)benzyl]-thiazolidine-2,4-dione (Compound No. 1-23)

0.4 g of acetic anhydride and 0.3 g of pyridine were added to a solution of 340 mg of 5-[4-(2,5-dihydroxy-3,4,6-trimethylphenoxy)benzyl]thiazolidine-2,4-dione (prepared as described in Example 4) in 6 ml of toluene, and the resulting mixture was stirred at room temperature for 3 days. At the end of this time, the reaction mixture was diluted with benzene, and the diluted mixture was washed with water. The mixture was dried over anhydrous sodium sulphate, after which the solvent was removed by distillation under reduced pressure. The resulting residue was purified by column chromatography through silica gel, using a 4 : 1 by volume mixture of benzene and ethyl acetate as the eluent, to give 340 mg of the title compound, melting at 174 - 176°C.

## EXAMPLES 8 TO 25

Following procedures similar to those described in Examples 1 to 7 above, we also prepared compounds of formula (I-4):

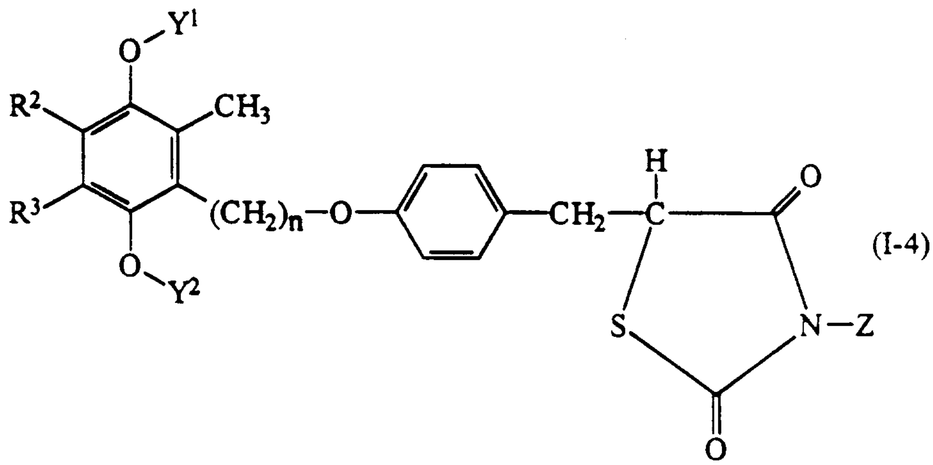

(I-4)

in which $R^2$, $R^3$, W and Z are as defined in Table 4. In the Table, the column "As in Ex. No." shows the number of the Example whose procedure was followed.

In this and subsequent Tables, the following abbreviations are used:

| | |
|---|---|
| Ac | = acetyl |
| Me | = methyl; |
| MeO | = methoxy; |
| Nic | = nicotinoyl |
| m.p. | = melting point |
| Ex. No. | = Example No. |
| Cpd. No. | = Compound No. (from the foregoing Tables 1 to 3) |
| (d) | is a decomposition point; and |
| (s) | is a softening point. |

Table 4

| Ex. No. | Cpd. No. | $R^2$ | $R^3$ | $Y^1$ | $Y^2$ | n | Z | As in Ex. No. | Property, m.p. (°C) |
|---|---|---|---|---|---|---|---|---|---|
| 8 | 1-13 | Me | Me | Me | Me | 1 | H | 3 | * 178 - 180 |
| 9 | 1-14 | Me | Me | Me | Me | 1 | Na | 6 | * white foamy powder |
| 10 | 1-18 | Me | Me | Me | Me | 3 | Na | 6 | 231 - 233 |
| 11 | 1-19 | Me | Me | Me | Me | 4 | H | 3 | 89 - 91 |
| 12 | 1-20 | Me | Me | Me | Me | 4 | Na | 6 | 235 - 239 |
| 13 | 1-44 | MeO | MeO | Me | Me | 1 | H | 3 | * white glassy powder |
| 14 | 1-45 | MeO | MeO | Me | Me | 1 | Na | 6 | * white foamy powder |
| 15 | 1-46 | MeO | MeO | Me | Me | 2 | H | 2 | * white foamy powder |
| 16 | 1-47 | MeO | MeO | Me | Me | 2 | Na | 6 | 181-185 |
| 17 | 1-48 | MeO | MeO | Me | Me | 3 | H | 3 | * pale yellow oil |
| 18 | 1-49 | MeO | MeO | Me | Me | 3 | Na | 6 | 204 - 206 |
| 19 | 1-50 | MeO | MeO | Me | Me | 4 | H | 3 | * colourless oil |
| 20 | 1-51 | MeO | MeO | Me | Me | 4 | Na | 6 | 215 - 217 |
| 21 | 1-27 | Me | Me | Ac | Ac | 2 | H | 4 & 7 | 122 - 125 |
| 22 | 1-29 | Me | Me | Ac | Ac | 3 | H | 4 & 7 | * white foamy powder |
| 23 | 1-30 | Me | Me | Ac | Ac | 3 | Na | 6 | 152 - 155 |
| 24 | 1-31 | Me | Me | Ac | Ac | 4 | H | 4 & 7 | * white foamy powder |
| 25 | 1-32 | Me | Me | Ac | Ac | 4 | Na | 6 | 205 - 209 |

* Nuclear Magnetic Resonance spectrum of the compound of Example 8 (δ ppm, $CDCl_3$):

2.20 (3H, singlet);
2.22 (3H, singlet);
2.29 (3H, singlet);
3.12 (1H, doublet of doublets, J = 9 & 14 Hz);
3.48 (1H, doublet of doublets, J = 4 & 14 Hz);
3.68 (3H, singlet);
3.69 (3H, singlet);
4.52 (1H, doublet of doublets, J = 4 & 9 Hz);
5.05 (2H, singlet);
6.98 (2H, doublet, J = 9 Hz);
7.17 (2H, doublet, J = 9 Hz);
8.14 (1H, broad singlet).

* Nuclear Magnetic Resonance spectrum of the compound of Example 9 is essentially identical to the Nuclear Magnetic Resonance spectrum of the compound of Example 8.

* Nuclear Magnetic Resonance spectrum of the compound of Example 13 (δ ppm, $CDCl_3$):

2.25 (3H, singlet);
3.13 (1H, doublet of doublets, J = 14 & 9 Hz);
3.48 (1H, doublet of doublets, J = 14 & 4 Hz);
3.81 (3H, singlet);
3.83 (1H, singlet);
3.92 (3H, singlet);
3.94 (3H, singlet);
4.52 (1H, doublet of doublets, J = 9 & 4 Hz);
5.01 (2H, singlet);
6.98 (2H, doublet, J = 9 Hz);
7.18 (2H, doublet, J = 9 Hz);
8.07 (1H, broad singlet).

* Nuclear Magnetic Resonance spectrum of the compound of Example 14 is essentially identical to the Nuclear

Magnetic Resonance spectrum of the compound of Example 13.

* Nuclear Magnetic Resonance spectrum of the compound of Example 15 (δ ppm, $CDCl_3$):

2.23 (3H, singlet);
3.0 - 3.2 (3H, multiplet);
3.44 (1H, doublet of doublets, J = 14 & 4 Hz);
3.79 (3H, singlet);
3.87 (3H, singlet);
3.91 (3H, singlet);
3.92 (3H, singlet);
4.03 (2H, triplet, J = 7 Hz);
4.50 (1H, doublet of doublets, J = 9 & 4 Hz);
6.87 (2H, doublet, J = 8 Hz);
7.13 (2H, doublet, J = 8 Hz);
8.14 (1H, broad singlet).

* Nuclear Magnetic Resonance spectrum of the compound of Example 17 (δ ppm, $CDCl_3$):

1.85 - 2.05 (2H, multiplet);
2.17 (3H, singlet);
2.76 (2H, triplet, J = 8 Hz);
3.11 (1H, doublet of doublets, J = 14 & 9 Hz);
3.45 (1H, doublet of doublets, J = 14 & 4 Hz);
3.78 (3H, singlet);
3.82 (3H, singlet);
3.89 (3H, singlet);
3.91 (3H, singlet);
3.99 (2H, triplet, J = 7 Hz);
4.50 (1H, doublet of doublets, J = 9 & 4 Hz);
6.85 (2H, doublet, J = 9 Hz);
7.14 (2H, doublet, J = 9 Hz);
8.30 (1H, broad singlet).

* Nuclear Magnetic Resonance spectrum of the compound of Example 19 (δ ppm, $CDCl_3$):

1.63 (2H, multiplet);
1.84 (2H, multiplet);
2.17 (3H, singlet);
2.64 (2H, triplet, J = 6 Hz);
3.10 (1H, doublet of doublets, J = 14 & 9 Hz);
3.44 (1H, doublet of doublets, J = 14 & 4 Hz);
3.78 (3H, singlet);
3.81 (3H, singlet);
3.89 (3H, singlet);
3.90 (3H, singlet);
3.98 (2H, triplet, J = 6 Hz);
4.50 (1H, doublet of doublets, J = 9 & 4 Hz);
6.84 (2H, doublet, J = 9 Hz);
7.13 (2H, doublet, J = 9 Hz);
7.92 (1H, broad singlet).

* Nuclear Magnetic Resonance spectrum of the compound of Example 22 (δ ppm, $CDCl_3$):

1.92 (2H, triplet, J = 6 Hz);
2.03 (3H, singlet);
2.05 (3H, singlet);
2.07 (3H, singlet);
2.30 (3H, singlet);
2.34 (3H, singlet);
2.69 (2H, multiplet);
3.14 (1H, doublet of doublets, J = 9 & 14 Hz);
3.45 (1H, doublet of doublets, J = 4 & 14 Hz);
3.94 (2H, triplet, J = 6 Hz);
4.51 (1H, doublet of doublets, J = 4 & 9 Hz);
6.84 (2H, doublet, J = 9 Hz);
7.14 (2H, doublet, J = 9 Hz);
7.83 (1H, broad singlet).

* Nuclear Magnetic Resonance spectrum of the compound of Example 24 (δ ppm, $CDCl_3$):

1.61 (2H, multiplet);
1.83 (2H, multiplet);
2.03 (3H, singlet);
2.05 (3H, singlet);
2.08 (3H, singlet);
2.29 (3H, singlet);
2.35 (3H, singlet);
2.55 (2H, multiplet);
3.11 (1H, doublet of doublets, J = 14 & 9 Hz);
3.45 (1H, doublet of doublets, J = 14 & 4 Hz);
3.95 (2H, triplet, J = 6 Hz);
4.50 (1H, doublet of doublets, J = 9 & 4 Hz);
6.83 (2H, doublet, J = 9 Hz);
7.13 (2H, doublet, J = 9 Hz);
7.99 (1H, broad singlet).

EXAMPLES 26 TO 29

Following procedures similar to those described in Examples 4, 6 and 7 above, we also prepared compounds of formula (I-5):

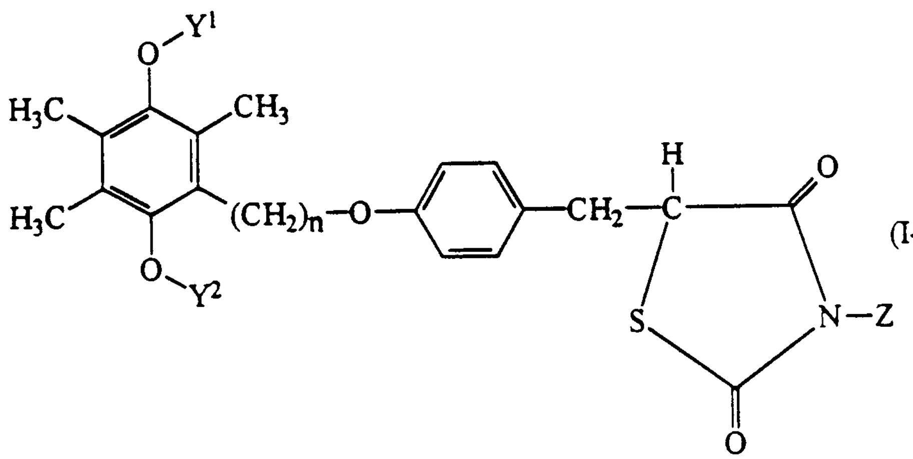

(I-5)

in which $Y^1$, $Y^2$, n and Z are as defined in Table 5. In the Table, the column "As in Ex. No." shows the number of the Example whose procedure was followed, and the abbreviations are as defined in relation to Table 4.

Table 5

| Ex. No. | Cpd. No. | $Y^1$ | $Y^2$ | n | Z | As in Ex. No. | Property, m.p. (°C) |
|---|---|---|---|---|---|---|---|
| 26 | 1-5 | H | H | 2 | H | 4 | 118 - 121 |
| 27 | 1-7 | H | H | 3 | H | 4 | * 116 - 120 |
| 28 | 1-28 | Ac | Ac | 2 | Na | 6 | * 265 - 268 (d) white powder |
| 29 | 1-65 | Nic | Nic | 3 | H | 7 | * 105 - 110 |

* Nuclear Magnetic Resonance spectrum of the compound of Example 27 (δ ppm, hexadeuterated dimethyl sulphoxide):

1.75 - 1.9 (2H, multiplet);
2.04 (6H, singlet);
2.06 (3H, singlet);
2.70 (2H, triplet, J = 8 Hz);
3.01 (1H, doublet of doublets, J = 9 & 14 Hz);
3.30 (1H, doublet of doublets, J = 4 & 14 Hz);
3.92 (2H, triplet, J = 6 Hz);
4.79 (1H, doublet of doublets, J = 4 & 9 Hz);
6.85 (2H, doublet, J = 8 Hz);
7.14 (2H, doublet, J = 8 Hz);
7.30 (1H, broad singlet, disappeared on adding $D_2O$);
7.32 (1H, broad singlet, disappeared on adding $D_2O$);
11.6 - 12.4 (1H, broad singlet, disappeared on adding $D_2O$).

* Nuclear Magnetic Resonance spectrum of the compound of Example 28 (δ ppm, hexadeuterated dimethyl sulphoxide):

1.98 (3H, singlet);
2.00 (3H, singlet);

2.10 (3H, singlet);
2.31 (3H, singlet);
2.36 (3H, singlet);
2.55 - 2.7 (1H, multiplet);
2.75 - 3.05 (2H, multiplet);
3.2 - 3.5 (1H, not determined);
3.9 - 4.05 (2H, multiplet);
4.05 - 4.15 (1H, multiplet);
6.78 (2H, doublet, J = 7 Hz);
7.09 (2H, doublet, J = 7 Hz).

* Nuclear Magnetic Resonance spectrum of the compound of Example 29 (δ ppm, hexadeuterated dimethyl sulphoxide):
1.7 - 2.0 (2H, multiplet);
2.05 (3H, singlet);
2.09 (3H, singlet);
2.13 (3H, singlet);
2.5 - 2.7 (1H, multiplet);
2.7 - 2.95 (1H, multiplet);
3.01 (1H, doublet of doublets, J = 9 & 14 Hz);
3.27 (1H, doublet of doublets, J = 4 & 14 Hz);
3.85-4.0 (2H, multiplet);
4.84 (1H, doublet of doublets, J = 4 & 9 Hz);
6.55 (2H, doublet, J = 9 Hz);
7.04 (2H, doublet, J = 9 Hz);
7.6 - 7.75 (2H, multiplet);
8.45 - 8.6 (2H, multiplet);
8.9 - 9.0 (2H, multiplet);
9.3 - 9.4 (2H, multiplet);
11.98 (1H, broad singlet).

EXAMPLES 30 TO 39

Following procedures similar to those described in Examples 2 to 7 above, we also prepared compounds of formula (I-6):

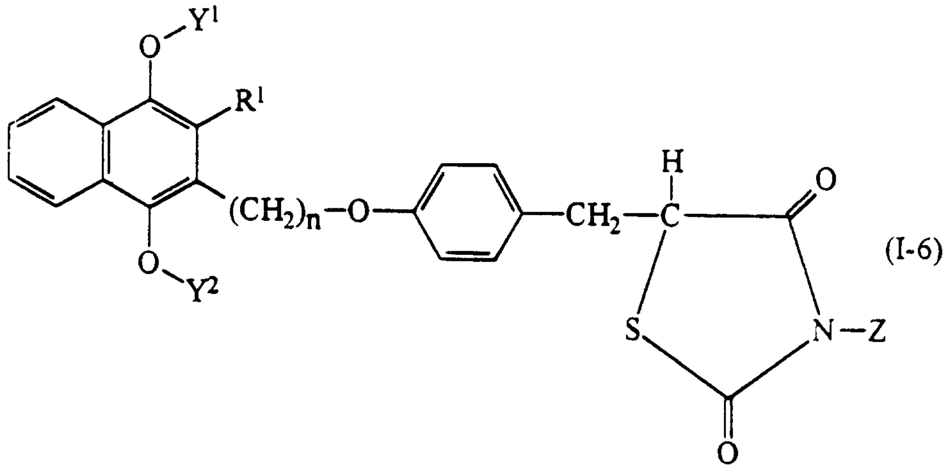

(I-6)

in which $R^1$, $Y^1$, $Y^2$, n and Z are as defined in Table 6. In the Table, the column "As in Ex. No." shows the number of the Example whose procedure was followed, and the abbreviations are as defined in relation to Table 4.

Table 6

| Ex. No. | Cpd. No. | $R^1$ | $Y^1$ | $Y^2$ | n | Z | As in Ex. No. | Property, m.p. (°C) |
|---|---|---|---|---|---|---|---|---|
| 30 | 2-7 | Cl | Ac | Ac | 0 | H | 7 | * 94 - 98 (s) |
| 31 | 2-4 | H | Me | Me | 1 | H | 3 | * 66 - 76 (s) pale yellow powder |
| 32 | 2-5 | H | Me | Me | 1 | Na | 6 | * 254 - 259 (d) pale yellow powder |
| 33 | 2-12 | Me | Me | Me | 1 | H | 3 | * 70 (s) pale yellow powder |
| 34 | 2-14 | H | Me | Me | 2 | H | 2 | * 60 - 65 (s) pale yellow powder |
| 35 | 2-15 | H | Me | Me | 2 | Na | 6 | * 240 - 250 (s) milky white powder |
| 36 | 2-16 | H | Me | Me | 3 | H | 2 | * 45 - 50 (s) pale yellow powder |
| 37 | 2-17 | H | Me | Me | 3 | Na | 6 | * 251 - 254 (d) white powder |
| 38 | 2-18 | H | Me | Me | 4 | H | 2 | * 37 - 42 (s) pale yellow powder |
| 39 | 2-19 | H | Me | Me | 4 | Na | 6 | * 261 - 265 white powder |

* Nuclear Magnetic Resonance spectrum of the compound of Example 30 (δ ppm, hexadeuterated dimethyl sulphoxide):

2.21 (3H, singlet);
2.55 (3H, singlet);
3.10 (1H, doublet of doublets, J = 9 & 14 Hz);
3.3 - 3.4 (1H, not determined);
4.89 (1H, doublet of doublets, J = 4 & 9 Hz);
6.84 (2H, doublet, J = 8 Hz);
7.21 (2H, doublet, J = 8 Hz);
7.67 - 7.75 (2H, multiplet);
7.95 - 8.1 (2H, multiplet);
12.03 (1H, broad singlet).

* Nuclear Magnetic Resonance spectrum of the compound of Example 31 (δ ppm, $CDCl_3$):

3.12 (1H, doublet of doublets, J = 9 & 14 Hz);
3.46 (1H, doublet of doublets, J = 4 & 14 Hz);
3.94 (3H, singlet);
3.98 (3H, singlet);
4.51 (1H, doublet of doublets, J = 4 & 9 Hz);
5.26 (2H, singlet);
6.87 (1H, singlet);
7.00 (2H, doublet, J = 9 Hz);
7.16 (2H, doublet, J = 9 Hz);
7.45 - 7.60 (2H, multiplet);
8.08 (1H, doublet, J = 9 Hz);
8.16 (1H, broad singlet);
8.24 (1H, doublet, J = 9 Hz).

* Nuclear Magnetic Resonance spectrum of the compound of Example 32 (δ ppm, hexadeuterated dimethyl sulphoxide):

2.71 (1H, doublet of doublets, J = 10 & 14 Hz);
3.33 (1H, doublet of doublets, J = 4 & 14 Hz);
3.87 (3H, singlet);
3.95 (3H, singlet);
4.22 (1H, doublet of doublets, J = 4 & 10 Hz);
5.20 (2H, singlet);

6.97 (1H, singlet);
7.00 (2H, doublet, J = 8 Hz);
7.15 (2H, doublet, J = 8 Hz);
7.55 (1H, triplet, J = 8 Hz);
7.61 (1H, triplet, J = 8 Hz);
8.04 (1H, doublet, J = 8 Hz);
8.16 (1H, doublet, J = 8 Hz).

* Nuclear Magnetic Resonance spectrum of the compound of Example 33 (δ ppm, $CDCl_3$):
2.46 (3H, singlet);
3.13 (1H, doublet of doublets, J = 9 & 14 Hz);
3.48 (1H, doublet of doublets, J = 4 & 14 Hz);
3.88 (3H, singlet);
3.95 (3H, singlet);
4.52 (1H, doublet of doublets, J = 4 & 9 Hz);
5.24 (2H, singlet);
7.03 (2H, doublet, J = 9 Hz);
7.20 (2H, doublet, J = 9 Hz);
7.45 - 7.58 (2H, multiplet);
8.07 - 8.16 (2H, multiplet);
8.42 (1H, broad singlet).

* Nuclear Magnetic Resonance spectrum of the compound of Example 34 (δ ppm, $CDCl_3$):
3.10 (1H, doublet of doublets, J = 14 & 9 Hz);
3.28 (2H, triplet, J = 7 Hz);
3.44 (1H, doublet of doublets, J = 14 & 4 Hz);
3.93 (3H, singlet);
3.98 (3H, singlet);
4.25 (2H, triplet, J = 7 Hz);
4.49 (1H, doublet of doublets, J = 9 & 4 Hz);
6.71 (1H, singlet);
6.88 (2H, doublet, J = 9 Hz);
7.13 (2H, doublet, J = 9 Hz);
7.42 - 7.58 (2H, multiplet);
7.99 - 8.12 (1H, broad singlet);

8.03 (1H, doublet, J = 8 Hz);
8.22 (1H, doublet, J = 8 Hz).

* Nuclear Magnetic Resonance spectrum of the compound of Example 35 (δ ppm, hexadeuterated dimethyl sulphoxide):

2.63 (1H, doublet of doublets, J = 10 & 14 Hz);
3.20 (2H, triplet, J = 7 Hz);
3.31 (1H, doublet of doublets, J = 4 & 14 Hz);
3.85 (3H, singlet);
3.94 (3H, singlet);
4.12 (1H, doublet of doublets, J = 4 & 14 Hz);
4.25 (2H, triplet, J = 7 Hz);
6.88 (2H, doublet, J = 9 Hz);
6.95 (1H, singlet);
7.10 (2H, doublet, J = 9 Hz);
7.48 (1H, triplet, J = 8 Hz);
7.57 (1H, triplet, J = 8 Hz);
7.98 (1H, doublet, J = 8 Hz);
8.11 (1H, doublet, J = 8 Hz).

* Nuclear Magnetic Resonance spectrum of the compound of Example 36 (δ ppm, $CDCl_3$):

2.12 - 2.25 (2H, multiplet);
2.99 (2H, triplet, J = 8 Hz);
3.10 (1H, doublet of doublets, J = 14 & 9 Hz);
3.45 (1H, doublet of doublets, J = 14 & 4 Hz);
3.88 (3H, singlet);
3.90 (3H, singlet);
4.01 (2H, triplet, J = 6 Hz);
4.50 (1H, doublet of doublets, J = 9 & 4 Hz);
6.61 (1H, singlet);
6.86 (2H, doublet, J = 9 Hz);
7.14 (2H, doublet, J = 9 Hz);
7.40 - 7.57 (2H, multiplet);
7.98 - 8.12 (1H, broad singlet);
8.02 (1H, doublet, J = 9 Hz);
8.20 (1H, doublet, J = 9 Hz).

* Nuclear Magnetic Resonance spectrum of the compound of Example 37 (δ ppm, hexadeuterated dimethyl sulphoxide):

2.05 - 2.14 (2H, multiplet);
2.63 (1H, doublet of doublets, J = 11 & 14 Hz);
2.91 (2H, triplet, J = 8 Hz);
3.31 (1H, doublet of doublets, J = 4 & 14 Hz);
3.80 (3H, singlet);
3.87 (3H, singlet);
4.00 (2H, triplet, J = 6 Hz);
4.11 (1H, doublet of doublets, J = 4 & 11 Hz);
6.80 (1H, singlet);
6.84 (2H, doublet, J = 9 Hz);
7.10 (2H, doublet, J = 9 Hz);
7.46 (1H, triplet, J = 8 Hz);
7.55 (1H, triplet, J = 8 Hz);
7.96 (1H, doublet, J = 8 Hz);
8.10 (1H, doublet, J = 8 Hz).

* Nuclear Magnetic Resonance spectrum of the compound of Example 38 (δ ppm, $CDCl_3$):

1.84 - 1.93 (4H, multiplet);
2.83 - 2.92 (2H, multiplet);
3.10 (1H, doublet of doublets, J = 9 & 14 Hz);
3.44 (1H, doublet of doublets, J = 4 & 14 Hz);
3.87 (3H, singlet);
3.97 (3H, singlet);
3.95 - 4.04 (2H, multiplet);
4.50 (1H, doublet of doublets, J = 4 & 9 Hz);
6.63 (1H, singlet);
6.84 (2H, doublet, J = 9 Hz);
7.12 (2H, doublet, J = 9 Hz);
7.41 - 7.55 (2H, multiplet);
7.88 (1H, broad singlet);
8.02 (1H, doublet, J = 9 Hz);
8.20 (1H, doublet, J = 9 Hz).

PREPARATION 1

Butyl 2-bromo-3-[4-(2,4,5-trimethyl-3,6-dimethoxyphenoxy)phenyl]pronionate

1(a) 2,5-Dimethoxy-3,4,6-trimethylphenol

A solution of 9.4 g of m-chloroperbenzoic acid (70% purity) in 100 ml of methylene chloride was added dropwise, whilst ice-cooling, to a solution of 4.6 g of 1,4-dimethoxy-2,3,5-trimethylbenzene in 20 ml of methylene chloride, and the resulting mixture was stirred at the same temperature for 30 minutes and then at room temperature for 5 hours. At the end of this time, the reaction mixture was washed with a 5% w/v aqueous solution of sodium hydrogensulphite, with a 5% w/v aqueous solution of sodium hydrogencarbonate and with water, in that order, after which it was dried over anhydrous sodium sulphate. The solvent was then removed from the reaction mixture by distillation under reduced pressure, and the resulting residue was purified by column chromatography through silica gel, using benzene and a 50 : 1 by volume mixture of benzene and ethyl acetate as the eluents, to give 1.3 g of the title compound.

Nuclear Magnetic Resonance Spectrum ($CDCl_3$) δ ppm:

2.12 (3H, singlet);
2.17 (6H, singlet);
3.65 (3H, singlet);
3.73 (3H, singlet);
5.59 (1H, singlet, disappeared on adding deuterium oxide).

1(b) 2,5-Dimethoxy-3,4,6-trimethyl-1-(4-nitrophenoxy)-benzene

5.8 g of 2,5-dimethoxy-3,4,6-trimethylphenol [prepared as described in step (a) above] in 10 ml of dimethylformamide were added to a suspension of 1.4 g of sodium hydride (as a 55% w/w dispersion in mineral oil) in 50 ml of dimethylformamide, whilst ice-cooling, and the mixture was stirred at room temperature for 2 hours. At the end of this time, a solution of 4.6 g of p-fluoronitrobenzene in 10 ml of dimethylformamide was added to the mixture, whilst ice-cooling. The mixture was then stirred at room temperature for 1 hour, and then at 80°C for 7 hours. At the end of this time, the mixture was poured into water, and the resulting crude oil was extracted with benzene. The benzene extract was washed with water and dried over anhydrous sodium sulphate. The solvent was then removed by distillation under reduced pressure, and the resulting oil was purified by column chromatography through silica gel, using a 4 : 1 by volume mixture of benzene and hexane, followed by benzene alone, as the eluent, to give 3.9 g of the title compound.

Nuclear Magnetic Resonance Spectrum ($CDCl_3$) δ ppm:

2.08 (3H, singlet);
2.19 (3H, singlet);
2.23 (3H, singlet);
3.65 (3H, singlet);
3.70 (3H, singlet);
6.89 (2H, doublet, J = 9 Hz);
8.17 (2H, doublet, J = 9 Hz).

1(c) 4-(2,5-Dimethoxy-3,4,6-trimethylphenoxy)aniline

A mixture of 4.8 g of 2,5-dimethoxy-3,4,6-trimethyl-1-(4-nitrophenoxy)benzene [prepared as described in step (b) above], 1.0 g of 10% w/w palladium-on-charcoal and 100 ml of ethanol was stirred under a hydrogen atmosphere at room temperature for 3 hours. At the end of this time, the catalyst was filtered off, and the filtrate was concentrated by evaporation under reduced pressure, to give 3.9 g of the title compound.

Nuclear Magnetic Resonance Spectrum ($CDCl_3$) δ ppm:

2.09 (3H, singlet);
2.17 (3H, singlet);
2.20 (3H, singlet);
3.4 (2H, broad singlet, disappeared on adding deutrium oxide);
3.667 (3H, singlet);
3.674 (3H, singlet);

6.59 (2H, doublet, J = 9 Hz);
6.65 (2H, doublet, J = 9 Hz).

1(d) Butyl 2-bromo-3-[4-(2,4,5-trimethyl-3,6-dimethoxyphenoxy)phenyl]propionate

7.7 g of a 47% w/v aqueous solution of hydrobromic acid and a solution of 1.3 g of sodium nitrite in 3 ml of water were added dropwise, in that order, to a solution of 4.3 g of 4-(2,5-dimethoxy-3,4,6-trimethylphenoxy)aniline [prepared as described in step (c) above] in 10 ml of acetone, after which 21 ml of butyl acrylate were added to the mixture. After that, 0.3 g of cupric bromide was gradually added and the resulting mixture was stirred at room temperature for 4 hours. At the end of this time, the reaction mixture was poured into water, after which it was extracted with benzene. The extract was washed with water and dried over anhydrous sodium sulphate. The solvent was removed by distillation under reduced pressure from the extract, and the residue thus obtained was purified by column chromatography through silica gel, using a 3 : 7 by volume mixture of hexane and benzene as the eluent, to give 5.7 g of the title compound.
Nuclear Magnetic Resonance Spectrum ($CDC\ell_3$) $\delta$ ppm:
0.87 (3H, singlet);
0.91 (3H, singlet);
0.93 (3H, singlet);
1.2 - 1.4 (2H, multiplet);
1.5 - 1.65 (2H, multiplet);
2.07 (3H, singlet);
2.17 (3H, singlet);
2.21 (3H, singlet);
3.16 (1H, doublet of doublets, J = 7 & 10 Hz);
3.39 (1H, doublet of doublets, J = 9 & 14 Hz);
3.65 (3H, singlet);
3.68 (3H, singlet);
4.11 (2H, triplet, J = 7 Hz);
4.33 (1H, doublet of doublets, J = 7 & 9 Hz);
6.73 (2H, doublet, J = 9 Hz);
7.08 (2H, doublet, J = 9 Hz).

PREPARATION 2

5-[4-(3,5,6-Trimethyl-1,4-benzoquinon-2-yloxy)-benzyl]thiazolidine-2,4-dione

A solution of 2.1 g of ceric ammonium nitrate in a mixture of 2 ml of water and 2 ml of acetonitrile was added dropwise at 0°C to a solution of 0.4 g of 5-[4-(2,4,5-trimethyl-3,6-dimethoxyphenoxy)benzyl]-thiazolidine-2,4-dione (prepared as described in Example 1) in 3 ml of acetonitrile, and the resulting mixture was stirred at the same temperature for 1 hour. At the end of this time, the reaction mixture was poured into water, after which it was extracted with ethyl acetate. The extract was washed with a saturated aqueous solution of sodium chloride and then dried over anhydrous sodium sulphate. The solvent was then removed from the extract by distillation under reduced pressure, and the residue thus obtained was purified by column chromatography through silica gel, using a 4 : 1 by volume mixture of benzene and ethyl acetate as the eluent, to give 260 mg of the title compound, melting at 153 - 156°C (with decomposition).

PREPARATION 3

5-{4-[2-(3,5,6-Trimethyl-1,4-benzoquinon-2-yl)-ethoxy]benzyl}thiazolidine-2,4-dione

Following a procedure similar to that described in Preparation 2, but using 5-{4-[2-(2,4,5-trimethyl-3,6-dimethoxyphenyl)ethoxy]benzyl}thiazolidine-2,4-dione (prepared as described in Example 2), the title compound, melting at 157 - 158°C, was obtained.

PREPARATION 4

5-{4-[3-(3,5,6-Trimethyl-1,4-benzoquinon-2-yl)-propoxy]benzyl}thiazolidine-2,4-dione

Following a procedure similar to that described in Preparation 2, but using 5-{4-[3-(2,4,5-trimethyl-3,6-dimethoxyphenyl)propoxy]benzyl}thiazolidine-2,4-dione (prepared as described in Example 10), the title compound, melting at 118 - 120 °C (with decomposition), was obtained.

PREPARATION 5

5-{4-[4-(3,5,6-Trimethyl-1,4-benzoquinon-2-yl)-butoxy]benzyl}thiazolidine-2,4-dione

Following a procedure similar to that described in Preparation 2, but using 5-{4-[4-(2,4,5-trimethyl-3,6-dimethoxyphenyl)butoxy]benzyl}thiazolidine-2,4-dione (prepared as described in Example 11), the title compound was obtained as a yellow foamy powder.
Nuclear Magnetic Resonance Spectrum ($CDC\ell_3$) δ ppm:

1.63 (2H, multiplet);
1.83 (2H, multiplet);
2.01 (6H, singlet);
2.03 (3H, singlet);
2.55 (2H, triplet, J = 7 Hz);
3.10 (1H, doublet of doublets, J = 9 & 14 Hz);
3.45 (1H, doublet of doublets, J = 4 & 14 Hz);
3.96 (2H, triplet, J = 6 Hz);
4.50 (1H, doublet of doublets, J = 4 & 9 Hz);
6.83 (2H, doublet, J = 9 Hz);
7.13 (2H, doublet, J = 9 Hz);
8.24 (1H, broad singlet).

PREPARATION 6

3-Chloro-2-(4-nitrophenoxy)-1,4-naphthoquinone

10 g of 2,3-dichlorol-1,4-naphthoquinone were added to a solution of 7 g of the sodium salt of p-nitrophenol in 100 ml of dimethylformamide, and the resulting mixture was stirred at room temperature for 5 hours. At the end of this time, the reaction mixture was poured into water, after which it was extracted with benzene. The extract was washed with water and dried over anhydrous sodium sulphate. The solvent was then removed from the extract by distillation under reduced pressure, and the residue thus obtained was purified by column chromatography through silica gel, using a 1 : 4 by volume mixture of hexane and benzene as the eluent, to give 10 g of the title compound, melting at 179 - 182 °C.

PREPARATION 7

Butyl 2-bromo-3-[4-(1,4-diacetoxy-3-chloro-2-naphthyloxy)phenyl]propionate

7(a) 3-Chlorol-1,4-dihydroxy-2-(4-nitrophenoxy)-naphthalene

1 g of sodium borohydride was added, whilst ice-cooling, to a solution of 11 g of 3-chloro-2-(4-nitrophenoxy)-1,4-naphthoquinone (prepared as described in Preparation 6) in 150 ml of methanol, and the mixture was stirred, whilst ice-cooling, for 30 minutes. The mixture was then poured into a mixture of ice and 15 ml of 2 N aqueous hydrochloric acid to give a precipitate, which was collected by filtration, washed with water and dried under reduced pressure in the presence of phosphorus pentoxide, to give 9 g of 3-chloro-1,4-dihydroxy-2-(4-nitrophenoxy)naphthalene.

7(b) 1,4-Diacetoxy-3-Chloro-2-(4-nitrophenoxy)-naphthalene

A mixture of the whole 9 g of this 3-chloro-1,4-dihydroxy-2-(4-nitrophenoxy)naphthalene [prepared as described in step (a) above], 6.6 g of acetic anhydride, 7 g of pyridine and 150 ml of benzene was then

stirred at room temperature for 20 hours. At the end of this time, the reaction mixture was poured into a mixture of ice and 15 ml of 2 N aqueous hydrochloric acid and extracted with benzene. The extract was washed with water and dried over anhydrous sodium sulphate. The solvent was then removed by distillation under reduced pressure, to give 7.8 g of 1,4-diacetoxy-3-chloro-2-(4-nitrophenoxy)naphthalene.

Thin layer chromatography:

Rf value: 0.40;

Adsorbent: silica gel plate No. 5715 (Merck);

Developing solvent: benzene.

7(c) 1,4-Diacetoxy-2-(4-aminophenoxy)-3-chloronaphthalene

Following a procedure similar to that described in Preparation 1(c), 8.5 g of the 1,4-diacetoxy-3-chloro-2-(4-nitrophenoxy)naphthalene [prepared as described in step (b) above] were hydrogenated under an atmosphere of hydrogen and in the presence of 1.7 g of 10% palladium-on-charcoal in 200 ml of tetrahydrofuran at room temperature for 5 hours, to give 8.3 g of 1,4-diacetoxy-2-(4-aminophenoxy)-3-chloronaphthalene as an oily substance.

Thin layer chromatography:

Rf value: 0.10;

Adsorbent: silica gel plate No. 5715 (Merck);

Developing solvent: a 10 : 0.3 by volume mixture of benzene and ethyl acetate.

7(d) Butyl 2-bromo-3-[4-(1,4-diacetoxy-3-chloro-2-naphthyloxy)phenyl]propionate

Following a procedure similar to that described in Preparation 1(d), 8.3 g of 1,4-diacetoxy-2-(4-aminophenoxy)-3-chloronaphthalene [prepared as described in step (c) above] were arylated using 15 g of a 47% w/v aqueous solution of hydrobromic acid, 1.9 g of sodium nitrate, 27 g of butyl acrylate and 0.5 g of cupric bromide, to give 5.8 g of the title compound as a pale yellow oil.

Nuclear Magnetic Resonance Spectrum ($CDC\ell_3$, partial) $\delta$ ppm:

0.91 (3H, triplet, J = 7 Hz);

3.19 (1H, doublet of doublets, J = 14 & 7 Hz);

3.41 (1H, doublet of doublets, J = 14 & 8 Hz);

4.34 (1H, doublet of doublets, J = 8 & 7 Hz).

PREPARATION 8

5-[4-(3-Chloro-1,4-naphthoquinon-2-yloxy)benzyl]-thiazolidine-2,4-dione

A mixture of 5.8 g of butyl 2-bromo-3-[4-(1,4-diacetoxy-3-chloro-2-naphthyloxy)phenyl]propionate (prepared as described in Preparation 7), 1 g of thiourea and 10 ml of sulpholane was heated at 120°C for 5 hours under an atmosphere of nitrogen. At the end of this time, 20 ml of ethylene glycol monomethyl ether and 10 ml of 2 N aqueous hydrochloric acid were added to the mixture in the presence of atmospheric oxygen, and the resulting mixture was heated at 100°C for 6 hours. The reaction mixture was then poured into water, after which it was extracted with benzene. The extract was washed with water and dried over anhydrous magnesium sulphate. The solvent was then removed from the extract by distillation under reduced pressure, and the resulting residue was purified by column chromatography through silica gel, using a 4 : 1 by volume mixture of benzene and ethyl acetate as the eluent. About 2.4g of the title compound were obtained by recrystallization from a mixture of tetrahydrofuran and hexane as crystals, melting at 250 - 252°C.

Nuclear Magnetic Resonance Spectrum (hexadeuterated dimethyl sulphoxide) $\delta$ ppm:

3.09 (1H, doublet of doublets, J = 14 & 9 Hz);

3.37 (1H, doublet of doublets, J = 14 & 4 Hz);

4.91 (1H, doublet of doublets, J = 9 & 4 Hz);

7.13 (2H, doublet, J = 8 Hz);

7.22 (2H, doublet, J = 8 Hz);

7.85 - 7.96 (2H, multiplet);

7.96 - 8.01 (1H, multiplet);

8.11 (1H, doublet, J = 7 Hz);

12.04 (1H, broad singlet, disappeared on adding deuterium oxide).

PREPARATION 9

5-{4-[3-(3,5,6-Trimethyl-1,4-benzoquinon-2-yl)-pronoxy]benzylidene}thiazolidine-2,4-dione

Following a procedure similar to that described in Preparation 2, but using 15.8 g of 5-{4-[3-(2,5-dimethoxy-3,4,6-trimethylphenyl)propoxy]benzyl}-thiazolidine-2,4-dione (prepared as described in Example 3), 78.1 g of ceric ammonium nitrate and 350 ml of acetonitrile, 1.7 g of the title compound, melting at 230 - 232 °C, were obtained.

Nuclear Magnetic Resonance Spectrum (hexadeuterated dimethyl sulphoxide) δ ppm:

1.80 - 1.87 (2H, multiplet);
1.92 (3H, singlet);
1.94 (6H, singlet);
2.60 (2H, triplet, J = 7 Hz);
4.04 (2H, triplet, J = 6 Hz);
7.04 (2H, doublet, J = 9 Hz);
7.53 (2H, doublet, J = 9 Hz);
7.77 (1H, singlet);
12.49 (1H, broad singlet).

PREPARATION 10

2-(2,3,4,5-Tetramethoxy-6-methylphenyl)ethanol

10(a) 1-Allyl-2,3,4,5-tetramethoxy-6-methylbenzene

A catalytic amount of iodine was added to a suspension of 975 mg of magnesium in 20 ml of tetrahydrofuran, and the resulting mixture was warmed up to about 45 °C to give rise to a white turbidity. A solution of 10.61 g of 2,3,4,5-tetramethoxy-6-methylbromobenzene in 30 ml of tetrahydrofuran was then added to the mixture, after which it was heated at about 45 °C for several minutes. The mixture was then stirred at room temperature for 30 minutes, after which 3.47 ml of allyl bromide were added dropwise to the mixture; it was then stirred at room temperature for 2 hours. At the end of this time, the reaction mixture was mixed with a saturated aqueous solution of ammonium chloride and then extracted with ethyl acetate. The solvent was removed from the extract by distillation under reduced pressure, and the residue thus obtained was purified by column chromatography through silica gel, using a 10 : 1 by volume mixture of hexane and ethyl acetate as the eluent, to give 7.98 g of the title compound as an oil.

Nuclear Magnetic Resonance Spectrum ($CDC\ell_3$) δ ppm: (only the signals due to an allyl group are reported) about 3.4 (2H, multiplet);

4.85 - 5.05 (2H, multiplet);
5.8 - 6.0 (1H, multiplet).

10(b) 2-(2,3,4,5-Tetramethoxy-6-methylphenyl)-acetaldehyde

109 mg of osmium tetroxide were added to a solution of 7.98 g of 1-allyl-2,3,4,5-tetramethoxy-6-methylbenzene [prepared as described in step (a) above] in a mixture of 300 ml of dioxane and 100 ml of water, and the resulting mixture was stirred at room temperature for 10 minutes. An aqueous solution of 35.6 g of sodium periodate was then added dropwise, and the mixture was stirred at room temperature for 2 hours. At the end of this time, the reaction mixture was freed from the dioxane by evaporation under reduced pressure, and the resulting concentrate was poured into a saturated aqueous solution of sodium chloride, after which it was extracted with diisopropyl ether. The solvent was then removed from the extract by distillation under reduced pressure, and the resulting residue was purified by column chromatography through silica gel, using a gradient elution method with mixtures of hexane and ethyl acetate ranging from 8 : 1 to 5 : 1 by volume as the eluent, to give 4.64 g of the title compound.

Nuclear Magnetic Resonance Spectrum ($CDC\ell_3$) (partial) δ ppm:

3.71 (2H, doublet, J = 2 Hz);
9.68 (1H, triplet, J = 2 Hz).

10(c) 2-(2,3,4,5-Tetramethoxy-6-methylphenyl)ethanol

5.38 g of 2-(2,3,4,5-tetramethoxy-6-methylphenyl)-acetaldehyde [prepared as described in step (b) above] were dissolved in 60 ml of ethanol and reduced using 400 mg of sodium borohydride at 0 °C. 150 ml of a saturated aqueous solution of sodium chloride were then added to the reaction mixture, and the mixture was extracted with ethyl acetate. The extract was dried over anhydrous magnesium sulphate and concentrated to dryness by evaporation under reduced pressure, to give a crude product. This crude product was then purified by column chromatography through silica gel, using a gradient elution method with mixtures of hexane and ethyl acetate ranging from 5 : 1 to 2 : 1 by volume as the eluent, to give 5.27 g of the title compound as a colourless oil.

Nuclear Magnetic Resonance Spectrum ($CDCl_3$) δ ppm:

2.19 (3H, singlet);
2.90 (2H, triplet, J = 7 Hz);
3.75 (2H, triplet, J = 7 Hz);
3.78 (3H, singlet);
3.85 (3H, singlet);
3.90 (3H, singlet);
3.91 (3H, singlet).

PREPARATION 11

1,4-Dimethoxy-2-naphthylmethanol

11 (a) Methyl 1,4-dimethoxy-2-naphthoate

20.7 g of anhydrous potassium carbonate were added to a solution of 5.1 g of 1,4-dihydroxy-2-naphthoic acid in 50 ml of dimethylformamide, and 28.4 g of methyl iodide were added dropwise to the resulting mixture, after which it was stirred for 19 hours. At the end of this time, the reaction mixture was poured into water, and the aqueous mixture was neutralized with 3 N aqueous hydrochloric acid and extracted with ethyl acetate. The extract was dried over anhydrous sodium sulphate, and the solvent was removed by distillation under reduced pressure. The resulting residue was purified by column chromatography through silica gel, using a 10 : 1 by volume mixture of hexane and ethyl acetate as the eluent, to give 5.45 g of the title compound as a yellow oil.

Thin layer chromatography:

Rf value: 0.24;
Adsorbent: silica gel plate No. 5715 (Merck);
Developing solvent: a 10 : 1 by volume mixture of hexane and ethyl acetate.

11(b) 1,4-Dimethoxy-2-naphthylmethanol

A solution of 5.32 g of methyl 1,4-dimethoxy-2-naphthoate [prepared as described in step (a) above] in 15 ml of tetrahydrofuran was added dropwise to a suspension of 0.98 g of lithium aluminium hydride in 15 ml of tetrahydrofuran, whilst ice-cooling. The resulting mixture was then stirred at room temperature for 1 hour, after which 20 ml of a saturated aqueous solution of ammonium chloride was added. The precipitate which formed was filtered off, and then the product was extracted with ethyl acetate. The extract was dried over anhydrous sodium sulphate and then concentrated by evaporation under reduced pressure, to give 3.97 g of the title compound as a pale yellow solid, melting at 63 - 66 °C.

Nuclear Magnetic Resonance Spectrum ($CDCl_3$) δ ppm:

3.92 (3H, singlet);
4.00 (3H, singlet);
4.89 (2H, singlet);
6.82 (1H, singlet);
7.45 - 7.6 (2H, multiplet);
8.04 (1H, doublet, J = 8 Hz);
8.23 (1H, doublet, J = 9 Hz).

PREPARATION 12

2-(1,4-Dimethoxy-2-naphthyl)ethanol

12(a) 1,4-Dimethoxy-2-naphthylmethyltriphenylphosphonium chloride

A solution of 4.73 g of 1,4-dimethoxy-2-naphthylmethyl chloride (prepared as described in Preparation 20) and 6.29 g of triphenylphosphine in 50 ml of dry acetonitrile was heated under reflux for 2 hours. At the end of this time, the reaction mixture was freed from the solvent by distillation under reduced pressure, and the resulting crystalline residue was washed with diethyl ether and air-dried, to give 7.36 g of the title compound as a white powder, melting at 244 - 246 °C (with decomposition).

12(b) 1,4-Dimethoxy-2-vinylnaphthalene

50 ml of a 10% aqueous solution of sodium hydroxide were added dropwise, with stirring, to a mixture of 7.36 g of 1,4-dimethoxy-2-naphthylmethyltriphenylphosphonium chloride [prepared as described in step (a) above] and 75 ml of a 30% v/v aqueous solution of formaldehyde, and the resulting mixture was stirred for 1 hour. At the end of this time, the reaction mixture was neutralized with 3 N aqueous hydrochloric acid, after which it was extracted with ethyl acetate. The extract was dried over anhydrous sodium sulphate, and the solvent was removed by distillation under reduced pressure. The resulting residue was purified by column chromatography through silica gel, using a 24 : 1 by volume mixture of hexane and ethyl acetate as the eluent, to give 2.45 g of the title compound as a pale yellow oil.
Thin layer chromatography:
Rf value: 0.53;
Adsorbent: silica gel plate No. 5715 (Merck);
Developing solvent: a 24 : 1 by volume mixture of hexane and ethyl acetate.

12(c) 2-(1,4-Dimethoxy-2-naphthyl)ethanol

1.61 g of titanium tetrachloride were added to a mixture of 0.65 g of sodium borohydride and 20 ml of dry ethylene glycol dimethyl ether, and the resulting mixture was stirred at room temperature for 1 hour. A solution of 1.83 g of 1,4-dimethoxy-2-vinylnaphthalene [prepared as described in step (b) above] in 40 ml of dry ethylene glycol dimethyl ether was then added dropwise to the resulting mixture, and the mixture was stirred for 21 hours. At the end of this time, the reaction mixture was poured into water, after which it was extracted with ethyl acetate. The extract was dried over anhydrous sodium sulphate, and the solvent was removed by distillation under reduced pressure. The resulting residue was purified by column chromatography through silica gel, using a 1 : 2 by volume mixture of hexane and ethyl acetate as the eluent, to give 0.40 g of the title compound as a colourless oil.
Nuclear Magnetic Resonance Spectrum ($CDC\ell_3$) $\delta$ ppm:
3.07 (2H, triplet, J = 7 Hz);
3.91 (3H, singlet);
3.93 (2H, triplet, J = 7 Hz);
3.98 (3H, singlet);
6.63 (1H, singlet);
7.4 - 7.6 (2H, multiplet);
8.02 (1H, doublet, J = 8 Hz);
8.22 (1H, doublet, J = 8 Hz).

PREPARATION 13

3-(1,4-Dimethoxy-2-naphthyl)propanol

13(a) 1,4-Dimethoxy-2-formylnaphthalene

4.18 g of manganese dioxide were added to a solution of 0.87 g of 1,4-dimethoxy-2-naphthylmethanol (prepared as described in Preparation 11) in 10 ml of methylene chloride, and the resulting mixture was stirred at room temperature for 6.5 hours. At the end of this time, the reaction mixture was filtered to remove inorganic materials, and the filtrate was dried over anhydrous sodium sulphate, after which the

solvent was removed by distillation under reduced pressure. The resulting crystalline residue was washed with hexane and air-dried, to give 0.57 g of the title compound as pale yellow needles, melting at 120 - 123 °C.

Thin layer chromatography:

Rf value: 0.44;

Adsorbent: silica gel plate No. 5715 (Merck);

Developing solvent: a 4 : 1 by volume mixture of hexane and ethyl acetate.

13(b) Methyl trans-3-(1,4-dimethoxy-2-naphthyl)acrylate

0.40 g of trimethyl phosphonoacetate was added to a suspension of 0.10 g of sodium hydride (as a 55% w/w dispersion in mineral oil, which had previously been washed with dry hexane) in 6 ml of dimethyl sulphoxide, and the resulting mixture was stirred for 20 minutes. 0.43 g of 1,4-dimethoxy-2-formylnaphthalene [prepared as described in step (a) above] was then added, whilst ice-cooling, to the mixture, and the mixture was stirred for 1 hour. At the end of this time, the reaction mixture was poured into water, after which it was extracted with ethyl acetate. The extract was dried over anhydrous sodium sulphate, and the solvent was removed by distillation under reduced pressure. The residue was purified by column chromatography through silica gel, using a 4 : 1 by volume mixture of hexane and ethyl acetate as the eluent, to give 0.47 g of the title compound as a pale yellow oil.

Thin layer chromatography:

Rf value: 0.42;

Adsorbent: silica gel plate No. 5715 (Merck);

Developing solvent: a 4 : 1 by volume mixture of hexane and ethyl acetate.

13(c) Methyl 3-(1,4-dimethoxy-2-naphthyl)propionate

0.47 g of methyl trans-3-(1,4-dimethoxy-2-naphthyl)-acrylate [prepared as described in step (b) above] was dissolved in 20 ml of methanol and hydrogenated under an atmosphere of hydrogen and in the presence of 0.20 g of 10% w/w palladium-on-charcoal, to give 0.41 g of the title compound as a colourless oil.

Thin layer chromatography:

Rf value: 0.66;

Adsorbent: silica gel plate No. 5715 (Merck);

Developing solvent: a 3 : 2 by volume mixture of hexane and ethyl acetate.

13(d) 3-(1,4-Dimethoxy-2-naphthyl)propanol

Following a procedure similar to that described in Preparation 11(b), but using 0.41 g of methyl 3-(1,4-dimethoxy-2-naphthyl)propionate [prepared as described in step (c) above], 68 mg of lithium aluminium hydride and 6 ml of tetrahydrofuran, 0.34 g of the title compound was obtained as a colourless oil.

Nuclear Magnetic Resonance Spectrum ($CDC\ell_3$) $\delta$ ppm:

1.85 - 2.0 (2H, multiplet);
2.91 (2H, triplet, J = 7 Hz);
3.58 (2H, triplet, J = 6 Hz);
3.91 (3H, singlet);
3.98 (3H, singlet);
6.60 (1H, singlet);
7.4 - 7.6 (2H, multiplet);
8.01 (1H, doublet, J = 8 Hz);
8.21 (1H, doublet, J = 8 Hz).

PREPARATION 14

4-(1,4-Dimethoxy-2-naphthyl)butanol

14(a) 4-(1,4-Dimethoxy-2-naphthyl)butyronitrile

A solution of 5.08 g of 3-(1,4-dimethoxy-2-naphthyl)propyl iodide (prepared as described in Preparation 21), and 0.70 g of sodium cyanide in 60 ml of dry dimethyl sulphoxide was stirred at 60°C (external temperature) for 80 minutes. At the end of this time, the reaction mixture was cooled and poured into water, after which it was extracted with ethyl acetate. The extract was dried over anhydrous sodium sulphate and the solvent was removed by distillation under reduced pressure. The resulting residue was purified by column chromatography through silica gel, using a 4 : 1 by volume mixture of hexane and ethyl acetate as the eluent, to give 3.36 g of the title compound as a colourless oil.
Thin layer chromatography:
Rf value: 0.19;
Adsorbent: silica gel plate No. 5715 (Merck);
Developing solvent: a 7 : 1 by volume mixture of hexane and ethyl acetate.

14(b) 4-(1,4-Dimethoxy-2-naphthyl)butyraldehyde

20 ml of a 1.0 M hexane solution of diisobutylaluminium hydride were added at -70°C to a solution of 3.36 g of 4-(1,4-dimethoxy-2-naphthyl)butyronitrile [prepared as described in step (a) above] in 100 ml of dry methylene chloride, and the resulting mixture was stirred for 2 hours. At the end of this time, water was added to the reaction mixture, and the insoluble materials were filtered off with the aid of a Celite (trade name) filter aid. The methylene chloride layer which separated was dried over anhydrous sodium sulphate, and the solvent was removed by distillation under reduced pressure, to give 2.96 g of the title compound as a colourless oil.
Thin layer chromatography:
Rf value: 0.19;
Adsorbent: silica gel plate No. 5715 (Merck);
Developing solvent: a 7 : 1 by volume mixture of hexane and ethyl acetate.

14(c) 4-(1,4-Dimethoxy-2-naphthyl)butanol

Following a procedure similar to that described in Preparation 1(c), but using 2.96 g of 4-(1,4-dimethoxy-2-naphthyl)butyraldehyde [prepared as described in step (b) above], 0.87 g of sodium borohydride and 80 ml of ethanol, 2.84 g of the title compound were obtained as a colourless oil.
Nuclear Magnetic Resonance Spectrum ($CDC\ell_3$) $\delta$ ppm:
1.6 - 1.95 (4H, multiplet);
2.83 (2H, triplet, J = 8 Hz);
3.71 (2H, triplet, J = 7 Hz);
3.87 (3H, singlet);
3.97 (3H, singlet);
6.61 (1H, singlet);
7.4 - 7.6 (2H, multiplet);
8.01 (1H, doublet, J = 8 Hz);
8.20 (1H, doublet, J = 8 Hz).

PREPARATION 15

3-(2,5-Dimethoxy-3,4,6-trimethylphenyl)propyl iodide

2.13 ml of methanesulphonyl chloride were added dropwise at 0°C to a mixture of 5.47 g of 3-(2,5-dimethoxy-3,4,6-trimethylphenyl)propanol, 4.8 ml of triethylamine and 50 ml of methylene chloride, and the resulting mixture was stirred for 30 minutes. At the end of this time, the reaction mixture was mixed with a mixture of 50 ml of ice-water and 50 ml of 10% w/v aqueous hydrochloric acid. The organic layer which separated was washed with a saturated aqueous solution of sodium hydrogencarbonate and with a saturated aqueous solution of sodium chloride, in that order, after which it was dried over anhydrous magnesium

sulphate. The solvent was then removed by distillation under reduced pressure, the residue was dissolved in 100 ml of acetone, and 6.88 g of sodium iodide were added to the resulting mixture. The reaction mixture was then stirred at 50°C for 2 hours, after which the solvent was removed by distillation under reduced pressure. The residue was mixed with 100 ml of a saturated aqueous solution of sodium thiosulphate, after which it was extracted with ethyl acetate. The extract was freed from the solvent by distillation under reduced pressure, and the residue was purified by column chromatography through silica gel, using a 10 : 1 by volume mixture of hexane and ethyl acetate as the eluent, to give 7.7 g of the title compound as an oil.

Nuclear Magnetic Resonance Spectrum ($CDCl_3$) δ ppm:

2.00 (2H, quintet, J = 7 Hz);
2.17 (6H, singlet);
2.23 (3H, singlet);
2.71 (2H, doublet of doublets, J = 7 Hz);
3.27 (2H, triplet, J = 7 Hz);
3.64 (3H, singlet);
3.67 (3H, singlet).

PREPARATIONS 16 to 22

Following a procedure similar to that described in Preparation 15 above, the following compounds of formula (I-7):

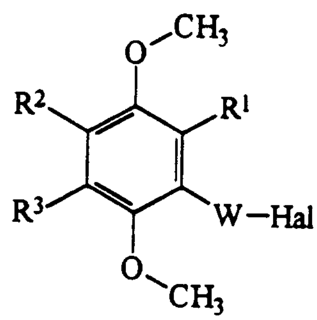

(I-7)

(in which $R^1$, $R^2$, $R^3$, W and Hal are as defined in Table 7) were obtained from the corresponding hydroxy compounds by replacing the hydroxy group of the hydroxy compound by the halogen atom shown in Table 7. The abbreviations are as defined in relation to Table 4. In Preparations 20, 21 and 22, $R^2$ and $R^3$ together represent the group shown under their columns.

Table 7

| Preparation No. | $R^1$ | $R^2$ | $R^3$ | W | Hal |
|---|---|---|---|---|---|
| 16 | Me | Me | Me | $-CH_2-$ | Br |
| 17 | Me | Me | Me | $-(CH_2)_4-$ | I |
| 18 | Me | MeO | MeO | $-CH_2-$ | Br |
| 19 | Me | MeO | MeO | $-(CH_2)_3-$ | I |
| 20 | H | -CH=CH-CH=CH- | | $-CH_2-$ | Cl |
| 21 | H | -CH=CH-CH=CH- | | $-(CH_2)_3-$ | I |
| 22 | Me | -CH=CH-CH=CH- | | $-CH_2-$ | Cl |

Nuclear Magnetic Resonance spectrum of the compound of Preparation 16, δ ppm, $CDCl_3$ (partial due to W):

4.66 (2H, singlet).

Nuclear Magnetic Resonance spectrum of the compound of Preparation 17, δ ppm, $CDCl_3$ (partial due to W):

1.50 - 1.70 (2H, multiplet);
1.85 - 2.00 (2H, multiplet);
2.63 (2H, doublet of doublets, J = 8 Hz);
3.24 (2H, triplet, J = 7 Hz).

Nuclear Magnetic Resonance spectrum of the compound of Preparation 18, δ ppm, $CDCl_3$ (partial due to W):
4.61 (2H, singlet).

Nuclear Magnetic Resonance spectrum of the compound of Preparation 19, δ ppm, $CDCl_3$ (partial due to W):
1.90 - 2.10 (2H, multiplet);
2.67 (2H, doublet of doublets, J = 8 Hz);
3.26 (2H, triplet, J = 7 Hz).

Nuclear Magnetic Resonance spectrum of the compound of Preparation 20, δ ppm, $CDCl_3$ (partial due to W):
4.85 (2H, multiplet).

Nuclear Magnetic Resonance spectrum of the compound of Preparation 21, δ ppm, $CDCl_3$ (partial due to W):
2.22 (2H, quintet, J = 7 Hz);
2.90 (2H, triplet, J = 7 Hz);
3.26 (2H, triplet, J = 7 Hz).

Nuclear Magnetic Resonance spectrum of the compound of Preparation 22, δ ppm, $CDCl_3$ (partial due to W):
4.92 (2H, singlet).

## PREPARATION 23

### 5-(4-Hydroxybenzyl)-3-triphenylmethyl-thiazolidine-2,4-dione

### 23(a) 5-(4-Acetoxybenzylidene)thiazolidine-2,4-dione

A mixture comprising 200 g of p-hydroxybenzaldehyde, 229 g of thiazolidine-2,4-dione, 280 g of sodium acetate and 660 ml of dimethylacetamide was stirred at 150° for 1 hour. It was then cooled, and 540 ml of dimethylacetamide and 370 ml of acetic anhydride were added to the reaction mixture. The resulting mixture was then stirred at 50°C for 1.5 hours, after which it was poured into water. The solid which precipitated was collected by filtration, washed with water, and dried in vacuo, to give 390 g of the title compound.

### 23(b) 5-(4-Acetoxybenzyl)thiazolidine-2,4-dione

2.0 g of 5-(4-acetoxybenzylidene)thiazolidine-2,4-dione [prepared as described in step (a) above] was dissolved in 80 ml of acetic acid and was hydrogenated under an atmosphere of hydrogen at atmospheric pressure at 90°C for 5 hours in the presence of 2.0 g of 10% w/w palladium-on-charcoal. At the end of this time, the catalyst was filtered off, and the filtrate was diluted with toluene. The acetic acid solvent was then removed by distillation as a toluene azeotrope. The crystals which separated out on adding toluene and hexane to the concentrate were collected by filtration and dried to give 1.8 g of the title compound.

### 23(c) 5-(4-Acetoxybenzyl)-3-triphenylmethyl-thiazolidine-2,4-dione

3.43 g of triethylamine were added to a solution of 9.0 g of 5-(4-acetoxybenzyl)thiazolidine-2,4-dione [prepared as described in step (b) above] in 70 ml of methylene chloride, and a solution of 9.45 g of triphenylmethyl chloride in 30 ml of methylene chloride was added dropwise to the resulting mixture. The mixture was then stirred at room temperature for 1 hour, after which it was allowed to stand overnight at the same temperature. At the end of this time, the reaction mixture was mixed with water and ethyl acetate, and the organic layer was separated, washed with a saturated aqueous solution of sodium chloride, and dried over anhydrous sodium sulphate. The crystals which separated out on distilling off the solvent under reduced pressure, were washed with a mixture of hexane and ethyl acetate and dried, to give 7.86 g of the title compound.

23(d) 5-(4-Hydroxybenzyl)-3-triphenylmethyl-thiazolidine-2,4-dione

A solution of 2.99 g of a 28% w/v methanolic solution of sodium methoxide in 10 ml of methanol was added dropwise, whilst ice-cooling, to a solution of 7.86 g of 5-(4-acetoxybenzyl)-3-triphenylmethyl-thiazolidine-2,4-dione [prepared as described in step (c) above] in 70 ml of toluene, and the resulting mixture was stirred at room temperature for 1 hour, after which it was allowed to stand overnight at the same temperature. The pH of the reaction mixture was then adjusted to a value of 4 by the addition of 1 N aqueous hydrochloric acid, and the mixture was extracted with ethyl acetate. The extract was washed with water and dried over anhydrous sodium sulphate. The solvent was then removed by distillation under reduced pressure, and the crystals which appeared in the residue were collected, washed with hexane and dried, to give 6.0 g of the title compound.

**Claims**

**1.** Compounds of formula (I):

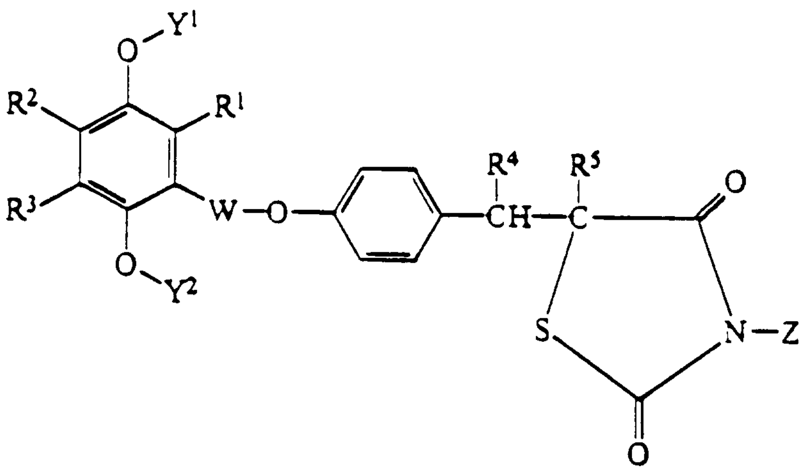

(I)

in which:

$R^1$ represents an alkyl group having from 1 to 5 carbon atoms;

$R^2$ and $R^3$ are the same or different and each represents an alkyl group having from 1 to 5 carbon atoms or an alkoxy group having from 1 to 5 carbon atoms,

or

$R^2$ and $R^3$ together form a benzene ring which is unsubstituted or which is substituted by at least one of substituents A, defined below, and, when $R^2$ and $R^3$ together form said benzene ring, $R^1$ represents a hydrogen atom, a halogen atom or an alkyl group having from 1 to 5 carbon atoms;

$R^4$ and $R^5$ both represent hydrogen atoms;

$Y^1$ and $Y^2$ are the same as each other or different from each other, and each represents:

a hydrogen atom,

an alkyl group having from 1 to 5 carbon atoms,

an aliphatic carboxylic acyl group having from 1 to 7 carbon atoms, or

a benzoyl, naphthoyl, pyridinecarbonyl or quinolinecarbonyl group which is unsubstituted or is substituted by at least one of substituents A, defined below;

W represents a single bond or an alkylene group having from 1 to 5 carbon atoms; and

Z represents a hydrogen atom or a 1/x equivalent of a cation, where x is the charge on the cation; and substituents A are selected from alkyl groups having from 1 to 5 carbon atoms, alkoxy groups having from 1 to 5 carbon atoms and halogen atoms.

**2.** A compound according to Claim 1, in which Z represents an alkali metal, one half equivalent of an alkaline earth metal or a basic amino acid.

**3.** A compound according to Claim 1 or Claim 2, in which $R^2$ and $R^3$ are the same.

4. A compound according to any one of Claims 1 to 3, in which $Y^1$ and $Y^2$ are the same and each represents a hydrogen atom, a methyl group, an acetyl group, a benzoyl group or a nicotinoyl group.

5. A compound according to any one of Claims 1 to 4, in which W represents an alkylene group having from 1 to 5 carbon atoms.

6. A compound according to any one of Claims 1 to 5, in which Z represents a hydrogen atom or a sodium atom.

7. A compound according to Claim 1, in which:
$R^1$ represents an alkyl group having from 1 to 5 carbon atoms;
$R^2$ and $R^3$ are the same or different and each represents an alkyl group having from 1 to 5 carbon atoms or an alkoxy group having 1 to 5 carbon atoms, or $R^2$ and $R^3$ together form a benzene ring which is unsubstituted or which is substituted by at least one of substituents A, defined above, and, when $R^2$ and $R^3$ together form said benzene ring, $R^1$ represents a hydrogen atom, a halogen atom or an alkyl group having from 1 to 5 carbon atoms;
$Y^1$ and $Y^2$ are the same and each represents a hydrogen atom, a methyl group, an acetyl group, a benzoyl group or a nicotinoyl group;
W represents an alkylene group having from 1 to 5 carbon atoms; and
Z represents a hydrogen atom or a sodium atom.

8. A compound according to Claim 1 or Claim 2, in which $R^2$ and $R^3$ are the same and each represents an alkyl group having from 1 to 5 carbon atoms, or $R^2$ and $R^3$ together form an unsubstituted benzene ring, and, when $R^2$ and $R^3$ together form said benzene ring, $R^1$ represents a hydrogen atom, a methyl group or a chlorine atom.

9. A compound according to Claim 8, in which $R^1$ represents a hydrogen atom.

10. A compound according to any one of Claims 1, 2, 8 and 9, in which $Y^1$ and $Y^2$ are the same and each represents a hydrogen atom, a methyl group or an acetyl group.

11. A compound according to Claim 10, in which $Y^1$ and $Y^2$ are the same and each represents a methyl group or an acetyl group.

12. A compound according to any one of Claims 1, 2, and 8 to 11, in which W represents an alkylene group having from 2 to 4 carbon atoms.

13. A compound according to Claim 1, in which:
$R^1$ represents an alkyl group having from 1 to 5 carbon atoms;
$R^2$ and $R^3$ are the same and each represents an alkyl group having from 1 to 5 carbon atoms, or $R^2$ and $R^3$ together form an unsubstituted benzene ring, and, when $R^2$ and $R^3$ together form said benzene ring, $R^1$ represents a hydrogen atom, a methyl group or a chlorine atom;
$Y^1$ and $Y^2$ are the same and each represents a hydrogen atom, a methyl group or an acetyl group;
W represents an alkylene group having from 2 to 4 carbon atoms; and
Z represents a hydrogen atom or a sodium atom.

14. A compound according to Claim 1 or Claim 2, in which $R^1$, $R^2$ and $R^3$ each represents a methyl group.

15. A compound according to any one of Claims 1, 2 and 14, in which W represents an ethylene or trimethylene group.

16. A compound according to Claim 1, in which:
$R^1$, $R^2$ and $R^3$ each represents a methyl group;
$Y^1$ and $Y^2$ are the same and each represents a methyl or acetyl group;
W represents an ethylene or trimethylene group; and
Z represents a hydrogen atom or a sodium atom.

**17.** The following compounds according to Claim 1:
5-{4-[3-(2,5-dihydroxy-3,4,6-trimethylphenyl)propoxy]-benzyl}thiazolidine-2,4-dione;
5-[4-(2,5-dimethoxy-3,4,6-trimethylbenzyloxy)benzyl]-thiazolidine-2,4-dione sodium salt;
5-{4-[3-(2,5-dimethoxy-3,4,6-trimethylphenyl)propoxy]-benzyl}thiazolidine-2,4-dione;
5-{4-[3-(2,5-dimethoxy-3,4,6-trimethylphenyl)propoxy]-benzyl}thiazolidine-2,4-dione sodium salt;
5-{4-[4-(2,5-dimethoxy-3,4,6-trimethylphenyl)butoxy]-benzyl}thiazolidine-2,4-dione sodium salt;
5-[4-(2,5-diacetoxy-3,4,6-trimethylphenoxy)benzyl]-thiazolidine-2,4-dione;
5-{4-[2-(2,5-diacetoxy-3,4,6-trimethylphenyl)ethoxy]-benzyl}thiazolidine-2,4-dione;
5-{4-[3-(2,5-diacetoxy-3,4,6-trimethylphenyl)propoxy]-benzyl}thiazolidine-2,4-dione sodium salt;
5-{4-[2-(2,3,4,5-tetramethoxy-6-methylphenyl)ethoxy]-benzyl}thiazolidine-2,4-dione sodium salt;
5-{4-[3-(2,3,4,5-tetramethoxy-6-methylphenyl)propoxy]-benzyl}thiazolidine-2,4-dione sodium salt;
5-{4-[4-(2,3,4,5-tetramethoxy-6-methylphenyl)butoxy]-benzyl}thiazolidine-2,4-dione;
5-{4-[4-(2,3,4,5-tetramethoxy-6-methylphenyl)butoxy]-benzyl}thiazolidine-2,4-dione sodium salt;
5-[4-(2,7-dimethoxynaphthylmethoxy)benzyl]thiazolidine-2,4-dione;
5-[4-(2,7-dimethoxynaphthylmethoxy)benzyl]thiazolidine-2,4-dione sodium salt;
5-[4-(2,7-dimethoxy-8-methylnaphthylmethoxy)benzyl]-thiazolidine-2,4-dione;
5-{4-[2-(2,7-dimethoxynaphthyl)ethoxy]benzyl}-thiazolidine-2,4-dione; and
5-{4-[2-(2,7-dimethoxynaphthyl)ethoxy]benzyl}-thiazolidine-2,4-dione sodium salt.

**18.** A pharmaceutical composition for the treatment or prophylaxis of diabetes or hyperlipemia, which comprises an active compound in admixture with a pharmaceutically acceptable carrier or diluent, in which said active compound is at least one compound of formula (I), as claimed in any one of Claims 1 to 17.

**19.** A compound of formula (I), as claimed in any one of Claims 1 to 18, for use in therapy.

**20.** The use of a compound of formula (I), as claimed in any one of Claims 1 to 17, for the manufacture of a medicament for the treatment or prophylaxis of diabetes or hyperlipemia in a mammal.

**21.** A process for preparing a compound according to any one of Claims 1 to 17, which comprises:
[a] reacting a compound of formula (II):

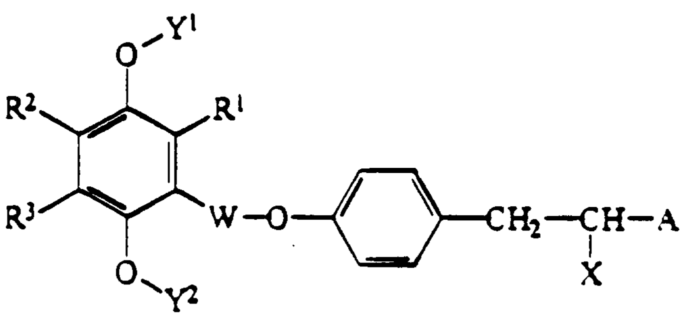

(II)

(in which $R^1$, $R^2$, $R^3$, $Y^1$, $Y^2$ and W are as defined in Claim 1; A represents a carboxyl, alkoxycarbonyl or carbamoyl group, or a group of formula -COOM, where M represents a cation; and X represents a halogen atom) with thiourea to produce an intermediate of formula (III):

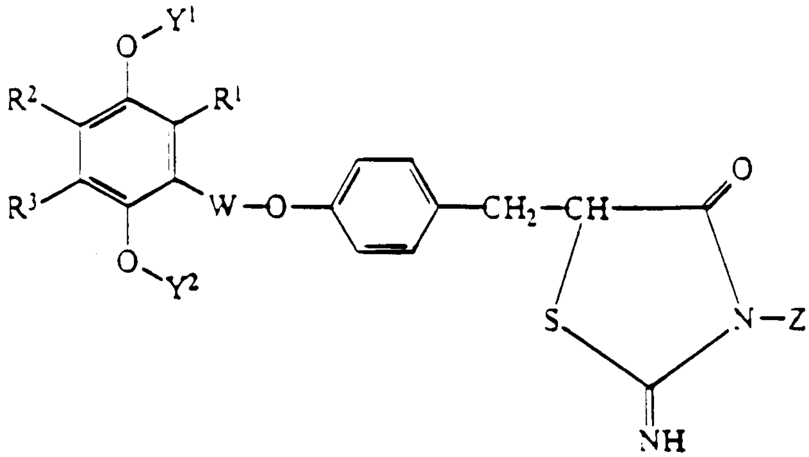

(III)

(in which $R^1$, $R^2$, $R^3$, $Y^1$, $Y^2$ and W are as defined above) and then hydrolysing the compound of formula (III);

or

[b] reacting a compound of formula (IV):

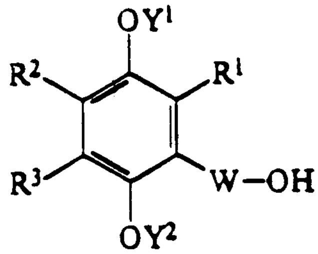

(IV)

(in which $R^1$, $R^2$, $R^3$, $Y^1$, $Y^2$ and W are as defined in Claim 1) or an active ester or halogenated derivative thereof with a compound of formula (V):

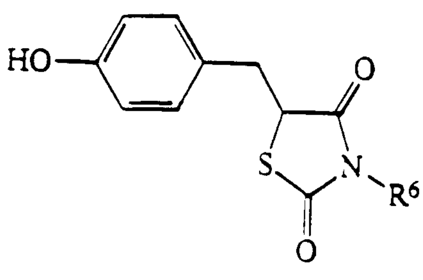

(V)

(in which $R^6$ represents a hydrogen atom or a protecting group) to give a compound of formula (VI):

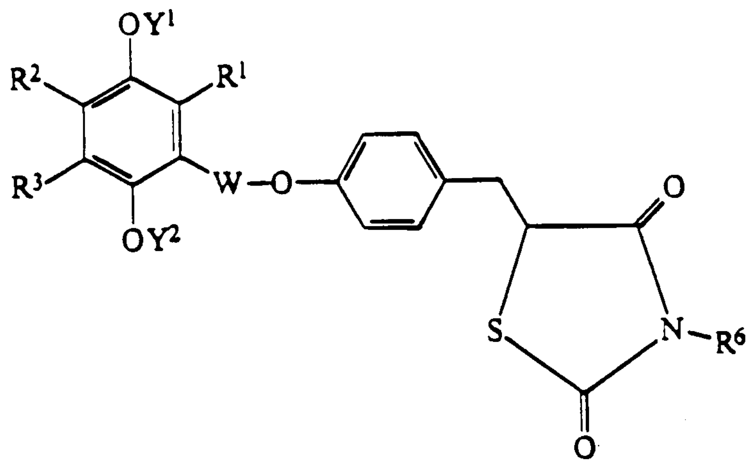

(VI)

(in which $R^1$, $R^2$, $R^3$, $R^6$, $Y^1$, $Y^2$ and W are as defined above), and, if necessary, removing the protecting group.

[c] oxidizing a compound of formula (X):

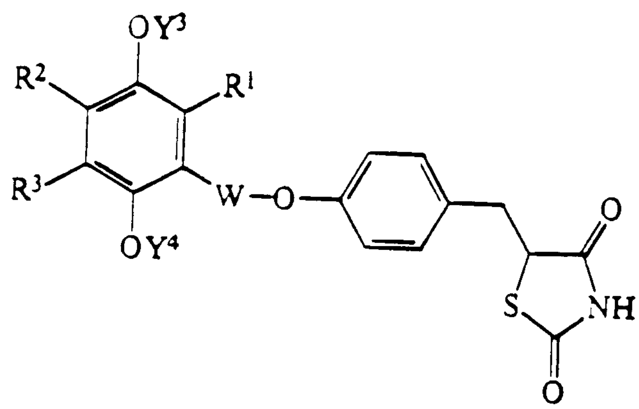

(X)

(in which $R^1$, $R^2$, $R^3$ and W are as defined in Claim 1, and $Y^3$ and $Y^4$ each represents an alkyl group), to give a compound of formula (XIII):

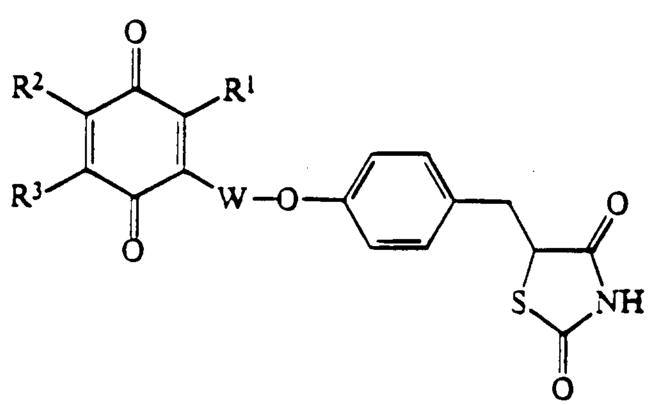

(XIII)

(in which $R^1$, $R^2$, $R^3$ and W are as defined above), and reducing said compound of formula (XIII), to give a compound of formula (XIV):

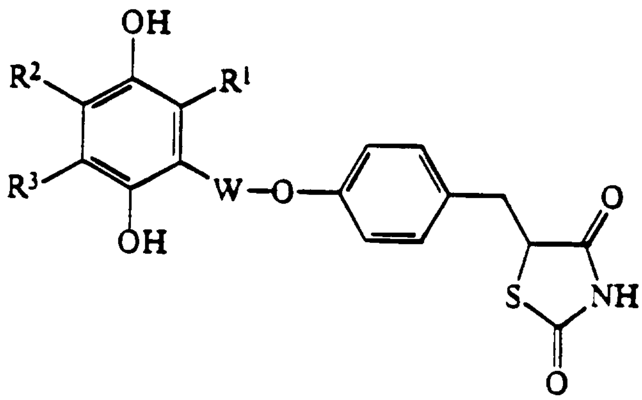

(XIV)

(in which $R^1$, $R^2$, $R^3$ and W are as defined above);

or

[d] reacting a compound of formula (XXI):

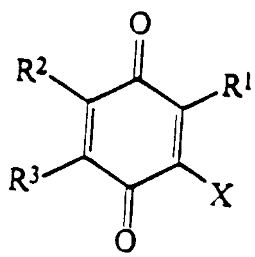

(XXI)

(in which $R^1$, $R^2$, $R^3$ are as defined in Claim 1 and X represents a halogen atom) with a compound of formula (XXII):

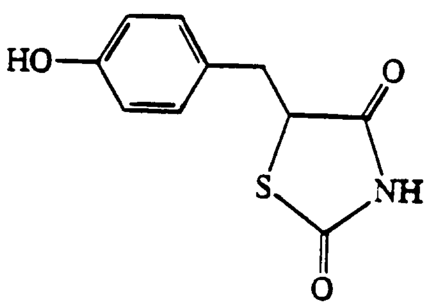

(XXII)

to give a compound of formula (XXIII):

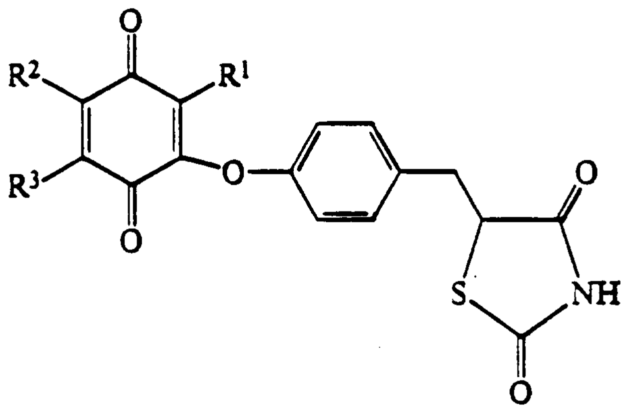

(XXIII)

(in which $R^1$, $R^2$, $R^3$ are as defined in Claim 1), and reducing, alkylating or acylating the compound of formula (XXIII), to give a compound of formula (I);

and

[e] optionally acylating the product of any preceding step where $Y^1$ and/or $Y^2$ represents a hydrogen atom, to give a compound of formula (I) in which $Y^1$ and/or $Y^2$ represents an acyl group;

and

[f] optionally salifying the product of any preceding step.

**Patentansprüche**

**1.** Verbindungen der Formel (I)

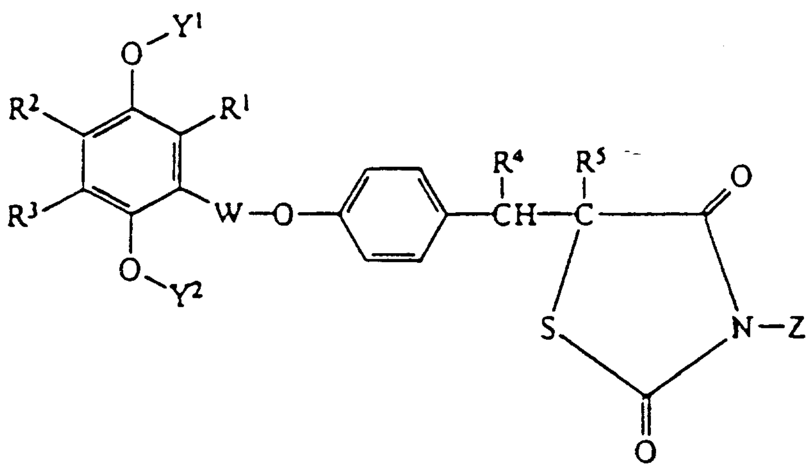

(I)

worin

$R^1$ eine Alkylgruppe mit 1 bis 5 Kohlenstoffatomen bedeutet,

$R^2$ und $R^3$ gleich oder verschieden sind und jeweils eine Alkylgruppe mit 1 bis 5 Kohlenstoffatomen oder eine Alkoxygruppe mit 1 bis 5 Kohlenstoffatomen bedeuten,

oder

$R^2$ und $R^3$ gemeinsam einen Benzolring bilden, der unsubstituiert ist oder der mit mindestens einem der nachstehend definierten Substituenten A substituiert ist und, wenn $R^2$ und $R^3$ zusammen diesen Benzolring bilden, $R^1$ ein Wasserstoffatom, ein Halogenatom oder eine Alkylgruppe mit 1 bis 5 Kohlenstoffatomen bedeutet,

beide Reste $R^4$ und $R^5$ Wasserstoffatome bedeuten,

$Y^1$ und $Y^2$ gleich oder verschieden voneinander sind und jeweils bedeuten:

ein Wasserstoffatom,

eine Alkylgruppe mit 1 bis 5 Kohlenstoffatomen,

eine Acylgruppe einer aliphatischen Carbonsäure mit 1 bis 7 Kohlenstoffatomen oder

eine Benzoyl-, Naphthoyl-, Pyridincarbonyl- oder Chinolincarbonylgruppe, die unsubstituiert ist oder die mit mindestens einem der nachstehend definierten Substituenten A substituiert ist,
W eine Einfachbindung oder eine Alkylengruppe mit 1 bis 5 Kohlenstoffatomen darstellt, und
Z ein Wasserstoffatom oder 1/x Äquivalent eines Kations bedeutet, wobei x die Ladung des Kations darstellt, und
die Substituenten A unter Alkylgruppen mit 1 bis 5 Kohlenstoffatomen, Alkoxygruppen mit 1 bis 5 Kohlenstoffatomen und Halogenatomen ausgewählt sind.

**2.** Verbindung nach Anspruch 1, worin Z ein Alkalimetall, ein halbes Äquivalent eines Erdalkalimetalls oder eine basische Aminosäure bedeutet.

**3.** Verbindung nach Anspruch 1 oder Anspruch 2, worin $R^2$ und $R^3$ gleich sind.

**4.** Verbindung nach einem der Ansprüche 1 bis 3, worin $Y^1$ und $Y^2$ gleich sind und jeweils ein Wasserstoffatom, eine Methylgruppe, eine Acetylgruppe, eine Benzoylgruppe oder eine Nicotinoylgruppe bedeuten.

**5.** Verbindung nach einem der Ansprüche 1 bis 4, worin W eine Alkylengruppe mit 1 bis 5 Kohlenstoffatomen bedeutet.

**6.** Verbindung nach einem der Ansprüche 1 bis 5, worin Z ein Wasserstoffatom oder ein Natriumatom bedeutet.

**7.** Verbindung nach Anspruch 1, worin:
$R^1$ eine Alkylgruppe mit 1 bis 5 Kohlenstoffatomen bedeutet,
$R^2$ und $R^3$ gleich oder verschieden sind und jeweils eine Alkylgruppe mit 1 bis 5 Kohlenstoffatomen oder eine Alkoxygruppe mit 1 bis 5 Kohlenstoffatomen bedeuten oder $R^2$ und $R^3$ gemeinsam einen Benzolring bilden, der unsubstituiert ist oder mit mindestens einem der vorstehend definierten Substituenten A substituiert ist, und, wenn $R^2$ und $R^3$ zusammen diesen Benzolring bilden, $R^1$ ein Wasserstoffatom, ein Halogenatom oder eine Alkylgruppe mit 1 bis 5 Kohlenstoffatomen bedeutet,
$Y^1$ und $Y^2$ gleich sind und jeweils ein Wasserstoffatom, eine Methylgruppe, eine Acetylgruppe, eine Benzoylgruppe oder eine Nicotinoylgruppe bedeuten,
W eine Alkylengruppe mit 1 bis 5 Kohlenstoffatomen bedeutet, und
Z ein Wasserstoffatom oder ein Natriumatom bedeutet.

**8.** Verbindung nach Anspruch 1 oder Anspruch 2, worin $R^2$ und $R^3$ gleich sind und jeweils eine Alkylgruppe mit 1 bis 5 Kohlenstoffatomen bedeuten oder $R^2$ und $R^3$ zusammen einen unsubstituierten Benzolring bilden, und, wenn $R^2$ und $R^3$ diesen Benzolring bilden, $R^1$ ein Wasserstoffatom, eine Methylgruppe oder ein Chloratom bedeutet.

**9.** Verbindung nach Anspruch 8, worin $R^1$ ein Wasserstoffatom bedeutet.

**10.** Verbindung nach einem der Ansprüche 1, 2, 8 und 9, worin $Y^1$ und $Y^2$ gleich sind und jeweils ein Wasserstoffatom, eine Methylgruppe oder eine Acetylgruppe bedeuten.

**11.** Verbindung nach Anspruch 10, worin $Y^1$ und $Y^2$ gleich sind und jeweils eine Methylgruppe oder eine Acetylgruppe bedeuten.

**12.** Verbindung nach einem der Ansprüche 1, 2 und 8 bis 11, worin W eine Alkylengruppe mit 2 bis 4 Kohlenstoffatomen bedeutet.

**13.** Verbindung nach Anspruch 1, worin:
$R^1$ eine Alkylgruppe mit 1 bis 5 Kohlenstoffatomen bedeutet,
$R^2$ und $R^3$ gleich sind und jeweils eine Alkylgruppe mit 1 bis 5 Kohlenstoffatomen bedeutet, oder $R^2$ und $R^3$ zusammen einen unsubstituierten Benzolring bilden, und, wenn $R^2$ und $R^3$ zusammen diesen Benzolring bilden, $R^1$ ein Wasserstoffatom, eine Methylgruppe oder ein Chloratom bedeutet,
$Y^1$ und $Y^2$ gleich sind und jeweils ein Wasserstoffatom, eine Methylgruppe oder eine Acetylgruppe bedeuten,

W eine Alkylengruppe mit 2 bis 4 Kohlenstoffatomen bedeutet, und
Z ein Wasserstoffatom oder ein Natriumatom bedeutet.

**14.** Verbindung nach Anspruch 1 oder Anspruch 2, worin $R^1$, $R^2$ und $R^3$ jeweils eine Methylgruppe bedeuten.

**15.** Verbindung nach einem der Ansprüche 1, 2 und 14, worin W eine Ethylen- oder Trimethylengruppe bedeutet.

**16.** Verbindung nach Anspruch 1, worin:
$R^1$, $R^2$ und $R^3$ jeweils eine Methylgruppe bedeuten,
$Y^1$ und $Y^2$ gleich sind und jeweils eine Methyl- oder Acetylgruppe bedeuten,
W eine Ethylen- oder Trimethylengruppe bedeutet und
Z ein Wasserstoffatom oder ein Natriumatom bedeutet.

**17.** Folgende Verbindungen gemäß Anspruch 1:
5-{4-[3-(2,5-Dihydroxy-3,4,6-trimethylphenyl)propoxy]benzyl}-thiazolidin-2,4-dion,
5-[4-(2,5-Dimethoxy-3,4,6-trimethylbenzyloxy)benzyl]-thiazolidin-2,4-dion-Natriumsalz,
5-{4-[3-(2,5-Dimethoxy-3,4,6-trimethylphenyl)propoxy]benzyl}-thiazolidin-2,4-dion,
5-{4-[3-(2,5-Dimethoxy-3,4,6-trimethylphenyl)propoxy]benzyl}-thiazolidin-2,4-dion-Natriumsalz,
5-{4-[4-(2,5-Dimethoxy-3,4,6-trimethylphenyl)butoxy]benzyl}-thiazolidin-2,4-dion-Natriumsalz,
5-[4-(2,5-Diacetoxy-3,4,6-trimethylphenoxy)benzyl]-thiazolidin-2,4-dion,
5-{4-[2-(2,5-Diacetoxy-3,4,6-trimethylphenyl)ethoxy]benzyl}-thiazolidin-2,4-dion,
5-{4-[3-(2,5-Diacetoxy-3,4,6-trimethylphenyl)propoxy]benzyl}-thiazolidin-2,4-dion-Natriumsalz,
5-{4-[2-(2,3,4,5-Tetramethoxy-6-methylphenyl)ethoxy]benzyl}-thiazolidin-2,4-dion-Natriumsalz,
5-{4-[3-(2,3,4,5-Tetramethoxy-6-methylphenyl)propoxy]benzyl}-thiazolidin-2,4-dion-Natriumsalz,
5-{4-[4-(2,3,4,5-Tetramethoxy-6-methylphenyl)butoxy]benzyl}-thiazolidin-2,4-dion,
5-{4-[4-(2,3,4,5-Tetramethoxy-6-methylphenyl)butoxy]benzyl}-thiazolidin-2,4-dion-Natriumsalz,
5-[4-(2,7-Dimethoxynaphthylmethoxy)benzyl]thiazolidin-2,4-dion,
5-[4-(2,7-Dimethoxynaphthylmethoxy)benzyl]thiazolidin-2,4-dion-Natriumsalz,
5-[4-(2,7-Dimethoxy-8-methylnaphthylmethoxy)benzyl]-thiazolidin-2,4-dion,
5-{4-[2-(2,7-Dimethoxynaphthyl)ethoxy]benzyl}thiazolidin-2,4-dion und
5-{4-[2-(2,7-Dimethoxynaphthyl)ethoxy]benzyl}thiazolidin-2,4-dion-Natriumsalz

**18.** Arzneimittelzusammensetzung zur Behandlung oder Prophylaxe von Diabetes oder Hyperlipämie, die eine aktive Verbindung im Gemisch mit einem pharmazeutisch geeigneten Trägermaterial oder Verdünnungsmittel enthält, wobei die aktive Verbindung mindestens eine Verbindung der Formel (I) gemäß einem der Ansprüche 1 bis 17 ist.

**19.** Verbindung der Formel (I) gemäß einem der Ansprüche 1 bis 18 zur therapeutischen Verwendung.

**20.** Verwendung einer Verbindung der Formel (I) gemäß einem der Ansprüche 1 bis 17 zur Herstellung eines Arzneimittels zur Behandlung oder Prophylaxe von Diabetes oder Hyperlipämie in einem Säuger.

**21.** Verfahren zur Herstellung einer Verbindung gemäß einem der Ansprüche 1 bis 17, welches umfaßt:

(a) Umsetzen einer Verbindung der Formel (II)

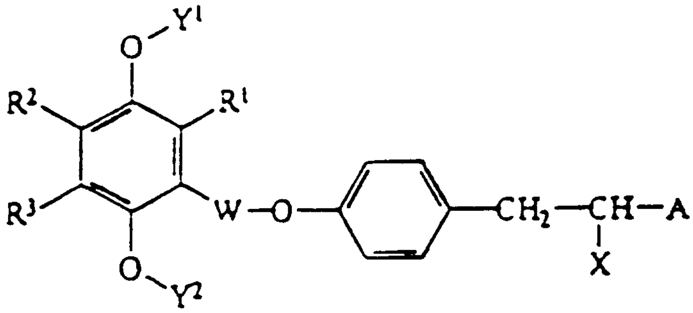

(II)

(worin $R^1$, $R^2$, $R^3$, $Y^1$, $Y^2$ und W wie in Anspruch 1 definiert sind, A eine Carboxy-, Alkoxycarbonyl- oder Carbamoylgruppe oder eine Gruppe der Formel -COOM bedeutet, wobei M ein Kation darstellt, und X ein Halogenatom bedeutet) mit Thioharnstoff unter Bildung eines Zwischenprodukts der Formel (III):

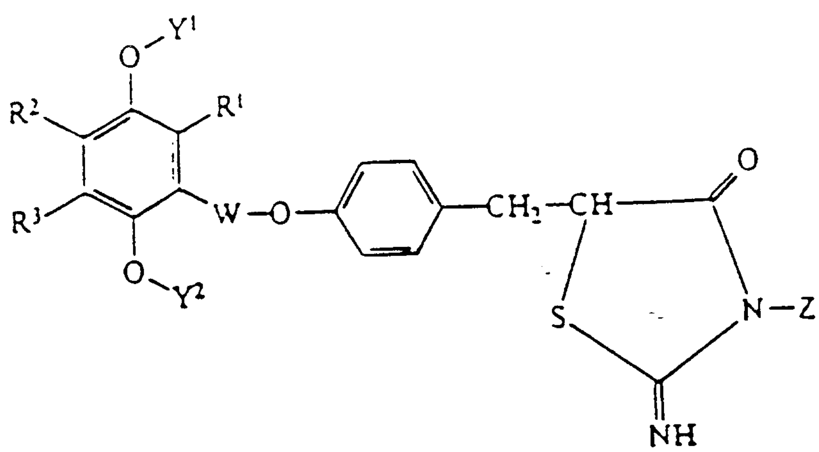

(III)

(worin $R^1$, $R^2$, $R^3$, $Y^1$, $Y^2$ und W wie oben definiert sind) und darauffolgende Hydrolyse der Verbindung der Formel (III) oder
(b) Umsetzen einer Verbindung der Formel (IV):

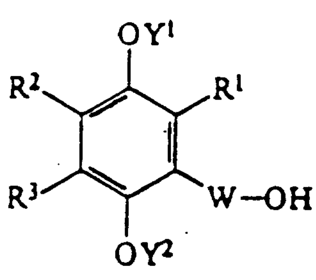

(IV)

(worin $R^1$, $R^2$, $R^3$, $Y^1$, $Y^2$ und W wie in Anspruch 1 definiert sind) oder eines aktiven Esters oder Halogenderivats dieser Verbindung mit einer Verbindung der Formel (V)

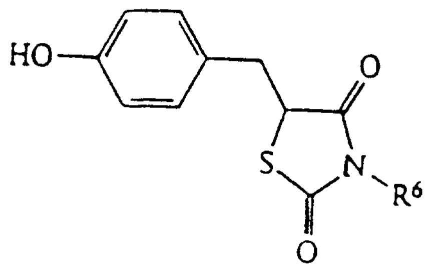

(V)

(worin $R^6$ ein Wasserstoffatom oder eine Schutzgruppe bedeutet) unter Bildung einer Verbindung der Formel (VI):

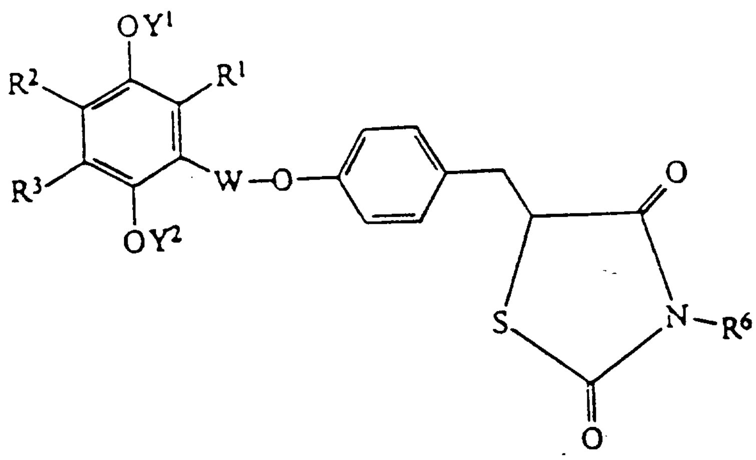

(VI)

(worin $R^1$, $R^2$, $R^3$, $R^6$, $Y^1$, $Y^2$ und W wie oben definiert sind) und erforderlichenfalls Entfernen der Schutzgruppe,
(c) Oxidation einer Verbindung der Formel (X):

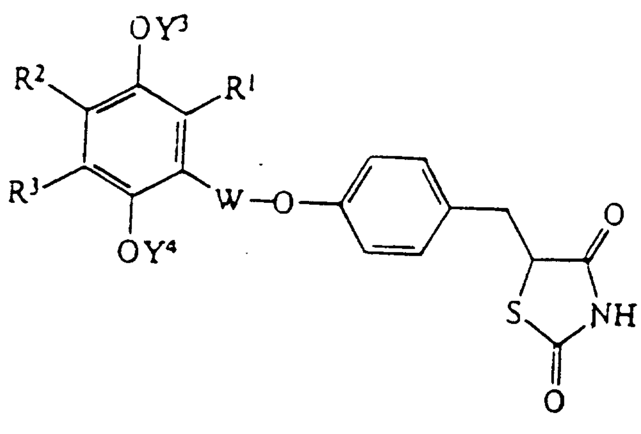

(X)

(worin $R^1$, $R^2$, $R^3$ und W wie in Anspruch 1 definiert sind und $Y^3$ und $Y^4$ jeweils eine Alkylgruppe bedeuten) unter Bildung einer Verbindung der Formel (XIII):

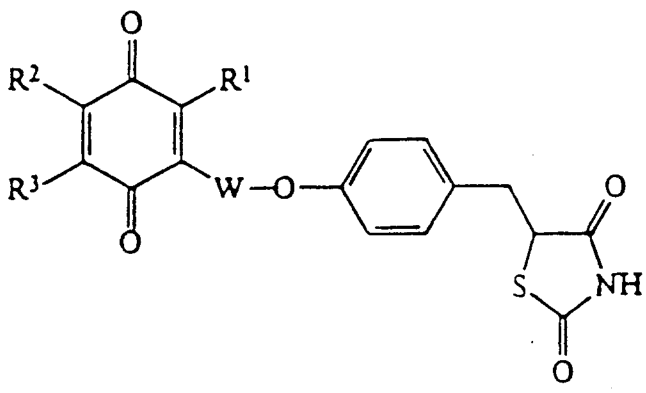

(XIII)

(worin $R^1$, $R^2$, $R^3$ und W wie oben definiert sind) und Reduktion dieser Verbindung der Formel (XIII) unter Bildung einer Verbindung der Formel (XIV):

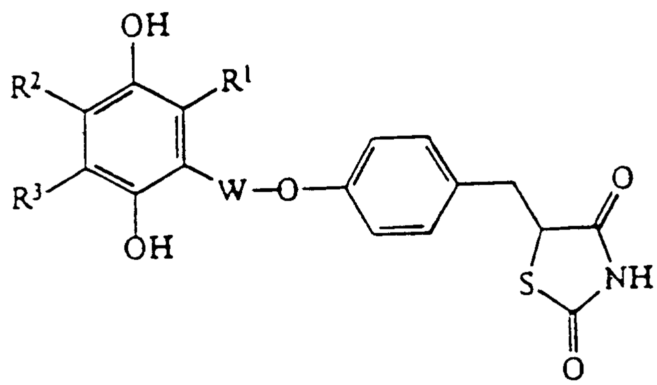

(XIV)

(worin $R^1$, $R^2$, $R^3$ und W wie oben definiert sind),
oder
(d) Umsetzen einer Verbindung der Formel der Formel (XXI):

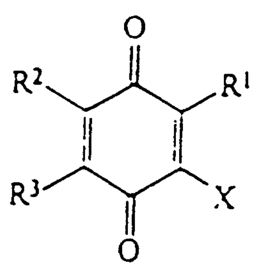

(XXI)

(worin $R^1$, $R^2$, $R^3$ wie in Anspruch 1 definiert sind und X ein Halogenatom bedeutet) mit einer Verbindung der Formel (XXII):

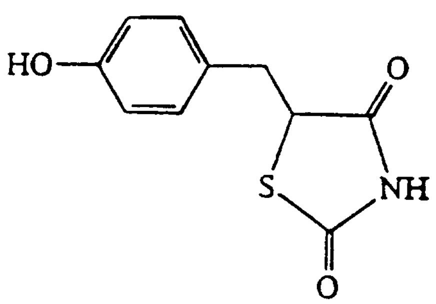

(XXII)

unter Bildung einer Verbindung der Formel (XXIII):

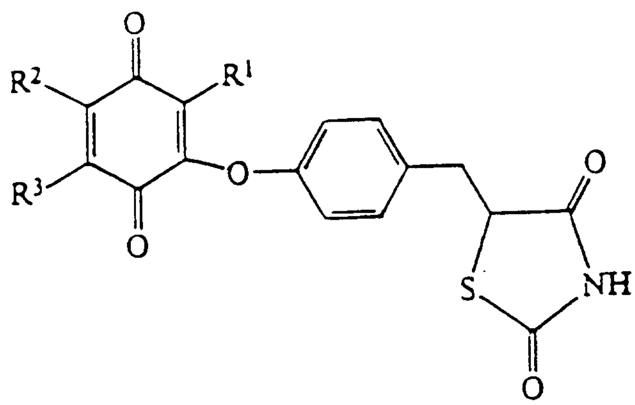

(XXIII)

(worin $R^1$, $R^2$, $R^3$ wie in Anspruch 1 definiert sind) und Reduktion, Alkylierung oder Acylierung der Verbindung der Formel (XXIII) unter Bildung einer Verbindung der Formel (I)
und
(e) gegebenenfalls Acylierung des Produkts aus irgendeiner vorhergehenden Stufe, wenn $Y^1$ und/oder $Y^2$ ein Wasserstoffatom bedeutet, unter Bildung einer Verbindung der Formel (I), in der $Y^1$ und/oder $Y^2$ eine Acylgruppe bedeutet,
und
(f) gegebenenfalls Überführen des Produkts aus irgendeiner der vorhergehenden Stufen in ein Salz.

**Revendications**

**1.** Composés de formule (I) :

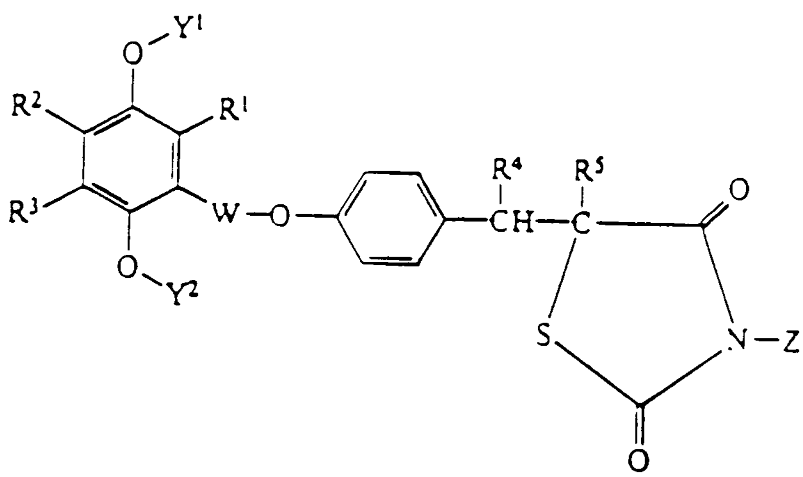

(I)

dans laquelle :
$R^1$ représente un groupe alkyle ayant 1 à 5 atomes de carbone ;
$R^2$ et $R^3$ sont identiques ou différents et représentent chacun un groupe alkyle ayant 1 à 5 atomes de carbone ou un groupe alcoxy ayant 1 à 5 atomes de carbone,
ou
$R^2$ et $R^3$, ensemble, forment un cycle benzène qui est non substitué ou qui est substitué par au moins un des substituants A définis ci-après, et, lorsque $R^2$ et $R^3$ forment ensemble ledit cycle benzène, $R^1$ représente un atome d'hydrogène, un atome d'halogène ou un groupe alkyle ayant 1 à 5 atomes de carbone ;
$R^4$ et $R^5$ représentent tous deux des atomes d'hydrogène ;
$Y^1$ et $Y^2$ sont identiques ou différents et représentent chacun :

un atome d'hydrogène,
un groupe alkyle ayant 1 à 5 atomes de carbone,
un groupe acyle carboxylique aliphatique ayant 1 à 7 atomes de carbone, ou
un groupe benzoyle, naphtoyle, pyridinecarbonyle ou quinoléinecarbonyle qui est non substitué ou est substitué par au moins un des substituants A définis ci-après ;
W représente une simple liaison ou un groupe alkylène ayant 1 à 5 atomes de carbone ; et
Z représente un atome d'hydrogène ou 1/x équivalent d'un cation, où x est la charge du cation ; et
les substituants A sont choisis parmi les groupes alkyle ayant 1 à 5 atomes de carbone, les groupes alcoxy ayant 1 à 5 atomes de carbone et les atomes d'halogène.

**2.** Composé selon la revendication 1, où Z représente un métal alcalin, un demi-équivalent d'un métal alcalino-terreux ou un amino-acide basique.

**3.** Composé selon la revendication 1 ou la revendication 2, où $R^2$ et $R^3$ sont identiques.

**4.** Composé selon l'une quelconque des revendications 1 à 3, où $Y^1$ et $Y^2$ sont identiques et représentent chacun un atome d'hydrogène, un groupe méthyle, un groupe acétyle, un groupe benzoyle ou un groupe nicotinoyle.

**5.** Composé selon l'une quelconque des revendications 1 à 4, où W représente un groupe alkylène ayant 1 à 5 atomes de carbone.

**6.** Composé selon l'une quelconque des revendications 1 à 5, où Z représente un atome d'hydrogène ou un atome de sodium.

**7.** Composé selon la revendication 1, où :
$R^1$ représente un groupe alkyle ayant 1 à 5 atomes de carbone ;
$R^2$ et $R^3$ sont identiques ou différents et représentent chacun un groupe alkyle ayant 1 à 5 atomes de carbone ou un groupe alcoxy ayant 1 à 5 atomes de carbone, ou
$R^2$ et $R^3$, ensemble, forment un cycle benzène qui est non substitué ou qui est substitué par au moins un des substituants A définis ci-après, et, lorsque $R^2$ et $R^3$ forment ensemble ledit cycle benzène, $R^1$ représente un atome d'hydrogène, un atome d'halogène ou un groupe alkyle ayant 1 à 5 atomes de carbone ;
$Y^1$ et $Y^2$ sont identiques et représentent chacun un atome d'hydrogène, un groupe méthyle, un groupe acétyle, un groupe benzoyle ou un groupe nicotinoyle ;
W représente un groupe alkylène ayant 1 à 5 atomes de carbone ; et
Z représente un atome d'hydrogène ou un atome de sodium.

**8.** Composé selon la revendication 1 ou la revendication 2, où $R^2$ et $R^3$ sont identiques et représentent chacun un groupe alkyle ayant 1 à 5 atomes de carbone, ou $R^2$ et $R^3$ forment ensemble un cycle benzène non substitué et, lorsque $R^2$ et $R^3$ forment ensemble ledit cycle benzène, $R^1$ représente un atome d'hydrogène, un groupe méthyle ou un atome de chlore.

**9.** Composé selon la revendication 8, où $R^1$ représente un atome d'hydrogène.

**10.** Composé selon l'une quelconque des revendications 1, 2, 8 et 9, où $Y^1$ et $Y^2$ sont identiques et représentent chacun un atome d'hydrogène, un groupe méthyle ou un groupe acétyle.

**11.** Composé selon la revendication 10, où $Y^1$ et $Y^2$ sont identiques et représentent chacun un groupe méthyle ou un groupe acétyle.

**12.** Composé selon l'une quelconque des revendications 1, 2 et 8 à 11, où W représente un groupe alkylène ayant 2 à 4 atomes de carbone.

**13.** Composé selon la revendication 1, ou :
$R^1$ représente un groupe alkyle ayant 1 à 5 atomes de carbone ; $R^2$ et $R^3$ sont identiques et représentent chacun un groupe alkyle ayant 1 à 5 atomes de carbone ou $R^2$ et $R^3$, ensemble, forment un cycle benzène non substitué et, lorsque $R^2$ et $R^3$ forment ensemble ledit cycle benzène, $R^1$

représente un atome d'hydrogène, un groupe méthyle ou un atome de chlore ; $Y^1$ et $Y^2$ sont identiques et représentent chacun un atome d'hydrogène, un groupe méthyle ou un groupe acétyle ;
W représente un groupe alkylène ayant 2 à 4 atomes de carbone ; et
Z représente un atome d'hydrogène ou un atome de sodium.

**14.** Composé selon la revendication 1 ou la revendication 2, où $R^1$, $R^2$ et $R^3$ représentent chacun un groupe méthyle.

**15.** Composé selon l'une quelconque des revendications 1, 2 et 14, où W représente un groupe éthylène ou triméthylène.

**16.** Composé selon la revendication 1, ou :
$R^1$, $R^2$ et $R^3$ représentent chacun un groupe méthyle ;
$Y^1$ et $Y^2$ sont identiques et représentent chacun un groupe méthyle ou acétyle ;
W représente un groupe éthylène ou triméthylène ; et
Z représente un atome d'hydrogène ou un atome de sodium.

**17.** Composés suivants selon la revendication 1 :
5-{4-[3-(2,5-dihydroxy-3,4,6-triméthylphényl)propoxy]benzyl} thiazolidine-2,4-dione ;
sel de sodium de la 5-[4-(2,5-diméthoxy-3,4,6-triméthylbenzyloxy) benzyl]thiazolidine-2,4-dione ;
5-{4-[3-(2,5-diméthoxy-3,4,6-triméthylphényl)propoxy]benzyl} thiazolidine-2,4-dione ;
sel de sodium de la 5-{4-[3-(2,5-diméthoxy-3,4,6-triméthylphényl) propoxy]benzyl}thiazolidine-2,4-dione ;
sel de sodium de la 5-{4-[4-(2,5-diméthoxy-3,4,6-triméthylphényl) butoxy]benzyl}thiazolidine-2,4-dione ;
5-[4-(2,5-diacétoxy-3,4,6-triméthylphénoxy)benzyl]thiazolidine-2,4-dione ;
5-{4-[2-(2,5-diacétoxy-3,4,6-triméthylphényl)éthoxy]benzyl} thiazolidine-2,4-dione ;
sel de sodium de la 5-{4-[3-(2,5-diacétoxy-3,4,6-triméthylphényl) propoxy]benzyl}thiazolidine-2,4-dione ;
sel de sodium de la 5-{4-[2-(2,3,4,5-tétraméthoxy-6-méthylphényl) éthoxy]benzyl}thiazolidine-2,4-dione ;
sel de sodium de la 5-{4-[3-(2,3,4,5-tétraméthoxy-6-méthylphényl) propoxy]benzyl}thiazolidine-2,4-dione ;
5-{4-[4-(2,3,4,5-tétraméthoxy-6-méthylphényl)butoxy]benzyl} thiazolidine-2,4-dione ;
sel de sodium de la 5-{4-[4-(2,3,4,5-tétraméthoxy-6-méthylphényl) butoxy]benzyl}thiazolidine-2,4-dione ;
5-[4-(2,7-diméthoxynaphtylméthoxy)benzyl]thiazolidine-2,4-dione ;
sel de sodium de la 5-[4-(2,7-diméthoxynaphtylméthoxy)benzyl] thiazolidine-2,4-dione ;
5-[4-(2,7-diméthoxy-8-méthylnaphtylméthoxy)benzyl]thiazolidine-2,4-dione ;
5-{4-[2-(2,7-diméthoxynaphtyl)éthoxy]benzyl}thiazolidine-2,4-dione ;
et
sel de sodium de la 5-{4-[2-(2,7-diméthoxynaphtyl)éthoxy]benzyl} thiazolidine-2,4-dione.

**18.** Composition pharmaceutique pour le traitement ou la prophylaxie du diabète ou de l'hyperlipémie, qui comprend un composé actif en mélange avec un véhicule ou diluant pharmaceutiquement acceptables, où ledit composé actif est au moins un composé de formule (I) selon l'une quelconque des revendications 1 à 17.

**19.** Composé de formule (I), selon l'une quelconque des revendications 1 à 18, pour l'utilisation en thérapeutique.

**20.** Utilisation d'un composé de formule (I), selon l'une quelconque des revendications 1 à 17, pour la fabrication d'un médicament pour le traitement ou la prophylaxie du diabète ou de l'hyperlipémie chez un mammifère.

**21.** Procédé pour préparer un composé selon l'une quelconque des revendications 1 à 17, qui comprend :

[a] la réaction d'un composé de formule (II) :

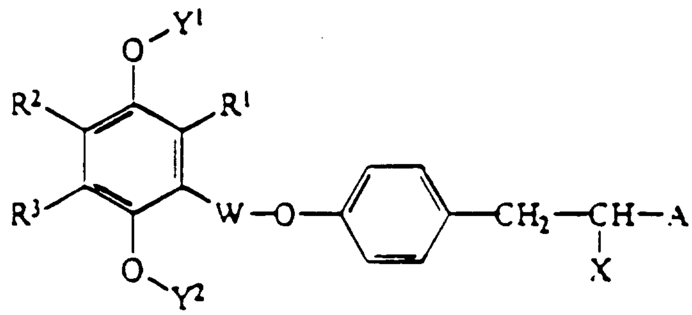

(II)

(dans laquelle $R^1$, $R^2$, $R^3$, $Y^1$, $Y^2$ et W sont tels que définis dans la revendication 1 ; A représente un groupe carboxy, alcoxycarbonyle ou carbamoyle, ou un groupe de formule -COOM, où M représente un cation ; et X représente un atome d'halogène) avec la thio-urée pour produire un intermédiaire de formule (III) :

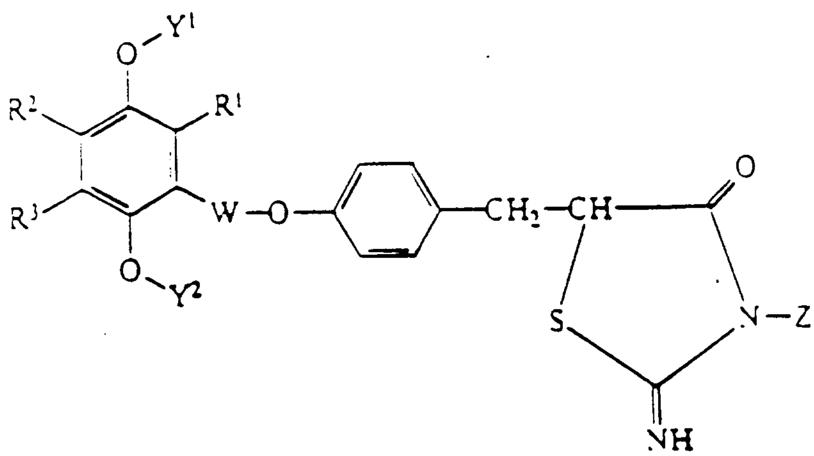

(III)

(dans laquelle $R^1$, $R^2$, $R^3$, $Y^1$, $Y^2$ et W sont tels que définis ci-dessus), puis l'hydrolyse du composé de formule (III) ;

ou

[b] la réaction d'un composé de formule (IV) :

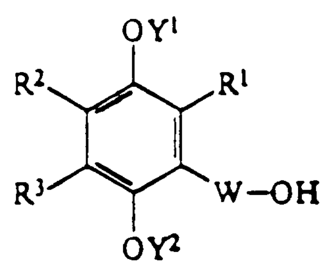

(IV)

(dans laquelle $R^1$, $R^2$, $R^3$, $Y^1$, $Y^2$ et W sont tels que définis dans la revendication 1) ou d'un ester actif ou d'un dérivé halogéné de celui-ci, avec un composé de formule (V) :

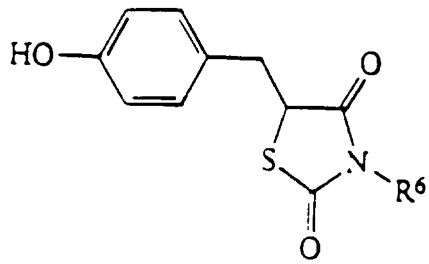

(V)

(dans laquelle $R^6$ représente un atome d'hydrogène ou un groupe protecteur) pour former un composé de formule (VI) :

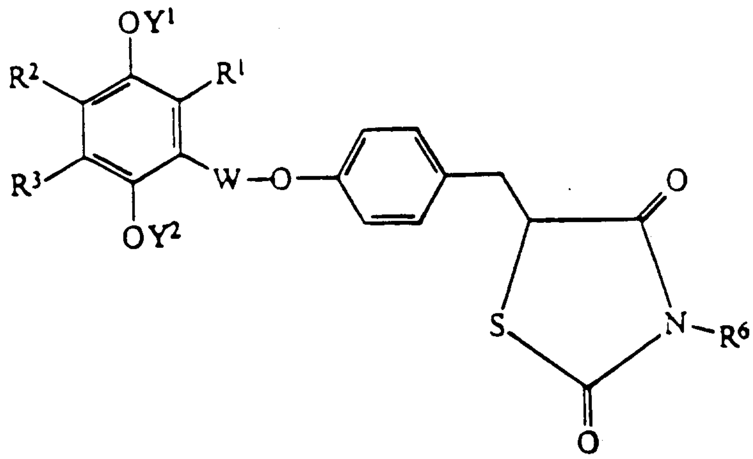

(VI)

(dans laquelle $R^1$, $R^2$, $R^3$, $R^6$, $Y^1$, $Y^2$ et W sont tels que définis ci-dessus) et, si besoin est, l'élimination du groupe protecteur ;

ou

[c] l'oxydation d'un composé de formule (X) :

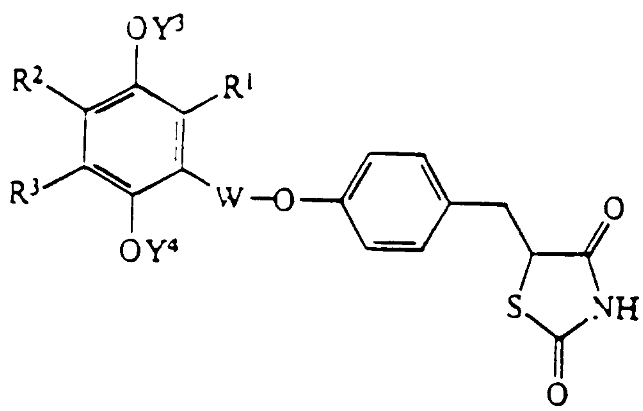

(X)

(dans laquelle $R^1$, $R^2$, $R^3$ et W sont tels que définis dans la revendication 1 et $Y^3$ et $Y^4$ représentent chacun un groupe alkyle) pour former un composé de formule (XIII) :

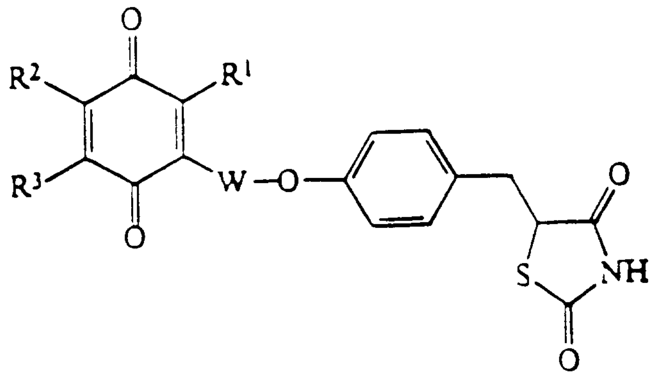

(XIII)

(dans laquelle $R^1$, $R^2$, $R^3$ et W sont tels que définis ci-dessus) et la réduction dudit composé de formule (XIII) pour obtenir un composé de formule (XIV) :

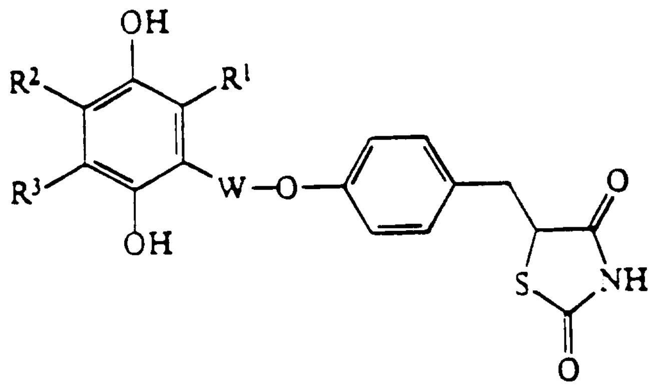

(XIV)

(dans laquelle $R^1$, $R^2$, $R^3$ et W sont tels que définis ci-dessus) ;

ou

[d] la réaction d'un composé de formule (XXI) :

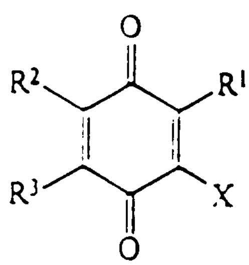

(XXI)

(dans laquelle $R^1$, $R^2$, $R^3$ sont tels que définis dans la revendication 1 et X représente un atome d'halogène) avec un composé de formule (XXII) :

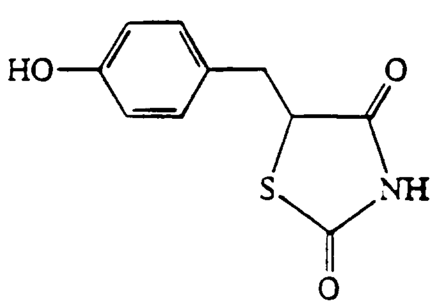

(XXII)

pour former un composé de formule (XXIII) :

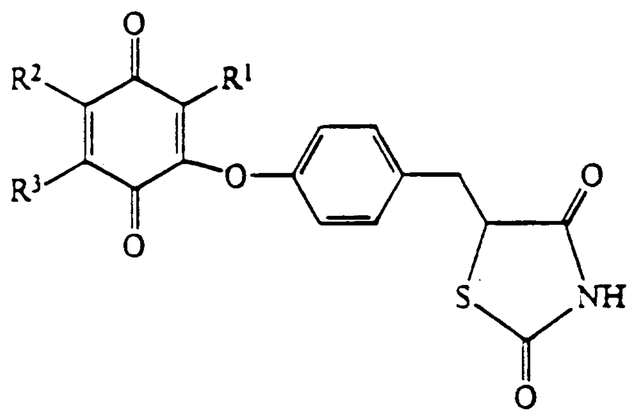

(XXIII)

(dans laquelle $R^1$, $R^2$ et $R^3$ sont tels que définis dans la revendication 1) et la réduction, l'alkylation ou l'acylation du composé de formule (XXIII) pour obtenir un composé de formule (I) ;

et

[e] facultativement, l'acylation du produit de l'une quelconque des étapes précédentes où $Y^1$ et/ou $Y^2$ représentent un atome d'hydrogène, pour former un composé de formule (I) dans laquelle $Y^1$ et/ou $Y^2$ représentent un groupe acyle ;

et

[f] facultativement, la salification du produit de l'une quelconque des étapes précédentes.